(19) Europäisches Patentamt
European Patent Office
Office européen des brevets

(11) EP 4 306 927 A1

(12) # EUROPEAN PATENT APPLICATION
published in accordance with Art. 153(4) EPC

(43) Date of publication:
**17.01.2024 Bulletin 2024/03**

(21) Application number: **22767290.4**

(22) Date of filing: **11.03.2022**

(51) International Patent Classification (IPC):
***G01N 1/30*** *(2006.01)*

(52) Cooperative Patent Classification (CPC):
**G01N 1/30**

(86) International application number:
**PCT/JP2022/011135**

(87) International publication number:
**WO 2022/191336 (15.09.2022 Gazette 2022/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.03.2021 US 202163160269 P**

(71) Applicant: **Kyoto University**
**Kyoto-shi, Kyoto 606-8501 (JP)**

(72) Inventors:
- **OGAWA, Takenobu**
  **Kyoto-shi, Kyoto 606-8501 (JP)**
- **MATSUMURA, Yasuki**
  **Kyoto-shi, Kyoto 606-8501 (JP)**

(74) Representative: **Hoffmann Eitle**
**Patent- und Rechtsanwälte PartmbB**
**Arabellastraße 30**
**81925 München (DE)**

(54) **TRANSPARENTIZING REAGENT AND TRANSPARENTIZING METHOD**

(57) Provided are a reagent for transparentizing a carbohydrate- or protein-containing material, the reagent including a compound selected from the group consisting of a benzene carboxylic acid compound, a hydroxybenzene compound, a benzene sulfonic acid compound, a homosulfamine compound, and a benzenesulfohydroxamic acid compound; and a method for transparentizing said material, including processing the carbohydrate- or protein-containing material in said reagent.

EP 4 306 927 A1

## Description

### Technical Field

The present invention relates to a reagent for optically clearing a carbohydrate- or protein-containing material, a method for optically clearing the material, a method for visualizing carbohydrates or proteins in the material, a kit and a device for use in the methods, and the like.

### Background Art

Gluten, which makes up 85% of endosperm wheat protein, is considered a crucial quality determinant of wheat-based food products. During wheat dough manufacture, the molecular packing of gluten causes formation of large structures that exceed the millimeter scale. However, despite numerous scientific studies that have been conducted on gluten over the past three centuries, the entire gluten structure remains unknown due to lack of imaging techniques for complex systems composed of giant macromolecules.

In recent years, a method for viewing 3D structures in biological samples such as mammal brains which involves mechanical sectioning and reconstruction has been developed. The said method comprises continuously slicing a sample with a microtome to obtain a set of continuous 2D images, and reconstructing these images in 3D using a computer software. Although this method enables access to deeper structures, it requires sophisticated techniques and labour and causes the destruction of microstructures in some cases. In contrast, optical sectioning using fluorescent labelling allows simple and fast imaging for 3D reconstruction. An obstacle facing deep imaging by optical sectioning is light scattering, which is caused by a mismatch in the refractive indices (RI) between sample components and surrounding solvents. Several optical clearing reagents and optically clearing methods have been developed to reduce the amount of light scattering in biological samples, such as mammal brains and plants (for example, see Patent Literature 1, Patent Literature 2, and Non-patent Literature 1). These reagents and methods aim to remove or denature the high-RI components, such as proteins, lipids and chlorophyll, and to replace the solvent with a high-RI reagent. For example, Patent Literature 1 discloses a method for optically clearing animal- or plant-derived biological materials, which comprises using a fructose-containing solution. Patent Literature 2 and Non-patent Literature 1 disclose optical clearing reagents for animal- or plant-derived biological materials, comprising urea as an effective ingredient. However, no studies aiming to optically clear food materials including wheat-based products have ever been reported.

### Citation List

#### Patent Literature

Patent Literature 1: JP Patent No. 5967528
Patent Literature 2: JP Patent No. 6044900

#### Non-patent Literature

Non-patent Literature 1: Kurihara D. et al., Development 142, 4168-4179 (2015)

### Summary of Invention

#### Problems to be solved by the Invention

The present inventors aimed to develop an optical clearing reagent for carbohydrate- or protein-containing materials including wheat-based products.

#### Solution to Problems

Wheat flour contains 80-85% starch, 8-14% proteins and 1% lipids, suggesting that starch will be a major source of light scattering in wheat-based samples. Therefore, the removal of starch or adjustment of the RI difference between starch and the surrounding substances was considered a potential approach to optically clear wheat-based samples. However, removal of starch would inevitably damage wheat-based samples because starch is present in wheat flour as large particles. Thus the present inventors tried to adjust the RI of the samples. As a result of extensive research, the

present inventors surprisingly found that treatment with certain aromatic compounds including benzenecarboxylic acid compounds made carbohydrate- or protein-containing materials, including wheat-based materials, transparent. Furthermore, in combination with fluorescent labeling, the inventors succeeded in visualizing and imaging the three-dimensional structure of carbohydrates or proteins in materials at the size in the millimeter scale and at submicron resolution. Thus the present invention was completed.

Specifically, the present disclosure provides the following aspects.

[1] A reagent for optically clearing a carbohydrate- or protein-containing material, comprising a compound selected from the group consisting of a benzenecarboxylic acid compound, a hydroxybenzene compound, a benzene sulfonic acid compound, a homosulfamine compound, and a benzenesulfohydroxamic acid compound.

[2] The reagent according to [1], wherein the reagent comprises a benzenecarboxylic acid compound, and the benzenecarboxylic acid compound is a compound represented by formula I:

[Chemical formula 1]

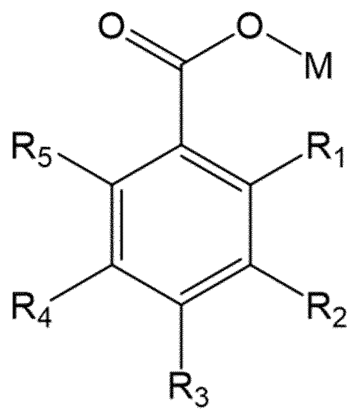

wherein,

$R_1$ to $R_5$ independently represent a hydrogen atom, a hydroxy group, an amino group, a nitro group, a carboxyl group, a C1-C4 alkyl group, a C2-C4 alkenyl group, a C2-C4 alkynyl group, a C1-C4 alkoxy group, or a halogen atom, wherein the C1-C4 alkyl group, the C2-C4 alkenyl group, the C2-C4 alkynyl group, and the C1-C4 alkoxy group may be optionally substituted with a halogen atom, and

M represents a monovalent or divalent metal atom or a hydrogen atom, and

when any of $R_1$ to $R_5$ is a carboxyl group, the carboxyl group may optionally form a salt together with a monovalent metal atom, or

when $R_1$ or $R_5$ is a carboxyl group and M is a divalent metal atom, the carboxyl group and M may optionally form a salt,

or a hydrate or hydrochloride thereof.

[3] The reagent according to [2], wherein one or two of $R_1$ to $R_5$ represent a hydroxy group.

[4] The reagent according to [2], wherein the benzenecarboxylic acid compound is a salt of benzoic acid, 2-, 3- or 4-hydroxybenzoic acid, or phthalic acid with a monovalent or divalent metal atom, or a hydrate thereof.

[5] The reagent according to any one of [2] to [4], wherein M is lithium, sodium, potassium, or calcium.

[6] The reagent according to [1], wherein the reagent comprises a hydroxybenzene compound, and the hydroxybenzene compound is a compound represented by formula II:

[Chemical formula 2]

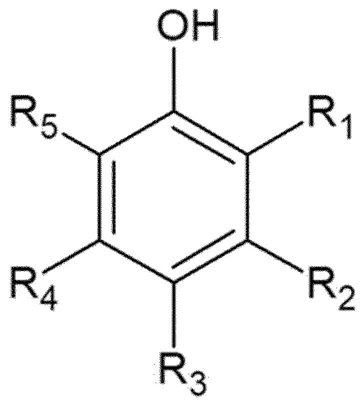

wherein,

$R_1$ to $R_5$ independently represent a hydrogen atom, a hydroxy group, an amino group, a nitro group, a sulfonic acid group, a sulfonamide group, an acetamide group, a 1-hydroxy-2-(methylamino)ethyl group, a methoxycarbonyl group, a C1-C4 alkyl group, a C2-C4 alkenyl group, a C2-C4 alkynyl group, a C1-C4 alkoxy group, or a halogen atom, wherein the C1-C4 alkyl group, the C2-C4 alkenyl group, the C2-C4 alkynyl group, and the C1-C4 alkoxy group may be optionally substituted with a halogen atom,

or a hydrate or hydrochloride thereof.

[7] The reagent according to [6], wherein one or two of $R_1$ to $R_5$ represent a hydroxy group.

[8] The reagent according to [6], wherein the hydroxybenzene compound is catechol, resorcinol, or pyrogallol, or a hydrate thereof.

[9] The reagent according to [1], wherein the reagent comprises a benzenesulfonic acid compound, and the benzenesulfonic acid compound is represented by formula III:

[Chemical formula 3]

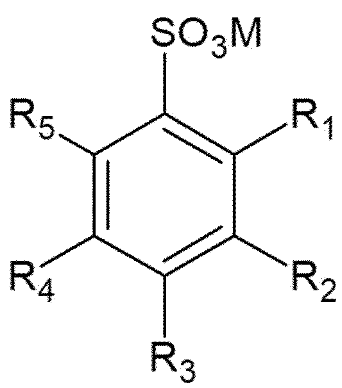

wherein,

$R_1$ to $R_5$ independently represent a hydrogen atom, a hydroxy group, a carboxyl group, an amino group, a nitro group, a sulfonic acid group, an aldehyde group, a C1-C4 alkyl group, a C2-C4 alkenyl group, a C2-C4 alkynyl group, a C1-C4 alkoxy group, or a halogen atom, wherein the C1-C4 alkyl group, the C2-C4 alkenyl group, the C2-C4 alkynyl group and the C1-C4 alkoxy group may be optionally substituted with a halogen atom, and

M represents a monovalent or divalent metal atom or a hydrogen atom, and

when any of $R_1$ to $R_5$ is a sulfonic acid group, the sulfonic acid group may optionally form a salt together with a monovalent metal atom, or

when $R_1$ or $R_5$ is a sulfonic acid group and M is a divalent metal atom, the sulfonic acid group and M may optionally form a salt,

or a hydrate or hydrochloride thereof.

[10] The reagent according to [9], wherein one or two of $R_1$ to $R_5$ represent a hydroxy group.
[11] The reagent according to [9] or [10], wherein M is lithium, sodium, potassium, calcium, or nickel.
[12] The reagent according to any one of [1] to [11], further comprising a fluorescent dye.
[13] A method of optically clearing a carbohydrate- or protein-containing material, the method comprising treating the carbohydrate- or protein-containing material with the reagent according to any one of [1] to [12].
[14] The method according to [13], which comprises treating the material with the reagent after defatting and/or decolorizing the material by pretreatment.
[15] The method according to [14], wherein the material is subjected to pretreatment with a decolorizing agent comprising 1,2-hexanediol and/or a defatting agent comprising limonene.
[16] A method of visualizing carbohydrates or proteins in a carbohydrate-containing material, the method comprising optically clearing the material by the method according to any one of [13] to [15].
[17] The method according to [16], further comprising treating the material with a fluorescent dye.
[18] The method according to [16] or [17], further comprising imaging.
[19] The method according to any one of [16] to [18], wherein the material is a wheat flour-based food material or a wheat flour processed food, and gluten protein is visualized.
[20] A kit for optically clearing a carbohydrate- or protein-containing material, comprising the reagent according to any one of [1] to [12].
[21] The kit according to [20], further comprising one or more selected from the group consisting of a fixative, a defatting agent, and a decolorizing agent.
[22] The kit according to [21], which comprises a decolorizing agent comprising 1,2-hexanediol and/or a defatting agent comprising limonene.
[23] A kit for visualizing carbohydrates or proteins in a material, comprising:

(a) the reagent according to any one of [1] to [11], and a fluorescent dye, or
(b) the reagent according to [12].

[24] The kit according to [23], further comprising one or more selected from the group consisting of a fixative, a defatting agent, and a decolorizing agent.
[25] The kit according to [24], which comprises a decolorizing agent comprising 1,2-hexanediol and/or a defatting agent comprising limonene.
[26] A device for visualizing carbohydrates or proteins in a material, comprising:

(a) the reagent according to any one of [1] to [11], a fluorescent dye a reagent, and an optical microscope, or
(b) the reagent according to [12], and an optical microscope.

[27] A cleared, carbohydrate- or protein-containing material, comprising the reagent according to any one of [1] to [12] .
[28] A decolorizing agent containing 1,2-hexanediol.
[29] A defatting agent containing limonene.

Effects of Invention

Carbohydrate- or protein-containing materials are easily and rapidly made transparent by using the optical clearing reagent or the optically clearing method of the present disclosure. Furthermore, according to the visualization method of the present disclosure, the structures of carbohydrates or proteins in carbohydrate- or protein-containing materials are visualized, and the structures can be observed by microscopy and/or imaging techniques. For example, it is possible to demonstrate how the three-dimensional structure of gluten changes from a honeycomb-shaped network to sparse large clumps in wheat noodles depending on salt added during dough making, thereby, for example, the texture of the noodles can also be changed.

The optical clearing reagent of the present disclosure can make carbohydrate- or protein-containing materials transparent in a few days to weeks, and enables visualization and imaging of the three-dimensional structures of carbohydrates or proteins in the materials at the size on a millimeter scale and at submicron resolution. An advantage of the optical clearing reagent of the present disclosure is that optical clearing is easily achieved only by immersion in the optical clearing reagent. Moreover, imaging devices for materials treated with the optical clearing regent of the present disclosure can be applied not only to a two-photon excitation microscopy (2PEM) but also to a confocal laser scanning microscopy (CLSM). Thus many researchers can easily access the technology of the present disclosure. In addition, optical sectioning which is applied to the optical clearing reagent and the optically clearing method of the present disclosure allows viewing of images of desired regions and planes in any orientation, which helps us to better understand

the structures (e.g. gluten structure) of materials.

Brief Description of Drawings

[FIG. 1-1] FIG. 1 shows optical clearing performance of SoROCS. Photographs (a-h) were obtained using ImageQuant for transmission. Scale bars indicate 5 mm. Untreated noodle sample (a) was fixed with 4% PFA/PBS for 1.5 hours (b) and immersed in water (0.5% (wt/wt) sodium azide added to prevent spoilage) (c, f), ClearSee (d, g), or SoROCS (e, h).

[FIG. 1-2] Opacity curves in the fixation process (light blue ◊) and clearing process using water (yellow □), ClearSee (orange ∆) or SoROCS (green o) were calculated from the transmission images of noodle samples without the grid pattern-printed film. Data are presented as mean values ±SD (N = 3 independently prepared noodle samples).

[FIG. 1-3] Photographs (j-l) were obtained using ImageQuant for transmission. The images presented in (j-l) are images of the same samples as in (f-h) in FIG. 1-2, respectively, with a grid pattern-printed film as a background. Scale bars indicate 5 mm. Plot profiles of the noodle samples treated with water (m), ClearSee (n) or SoROCS (o) are provided. The red line in (j-l) indicates the corresponding location of the plot profile in (m-o), respectively. The contrast value in the plot profile for the areas shown in curly brackets is shown in (m-o).

[FIG. 2-1] FIG. 2 shows expansion characterisation during clearing. Linear expansion curves in fixation process (light blue ◊) and clearing process using water (yellow □) or SoROCS (green o) were calculated. Data are presented as mean values ± SD (N = 3 independently prepared noodle samples).

[FIG. 2-2] (b) shows CLSM images of gluten in a non-cleared noodle sample section, which was mechanically sliced using a cryostat. (c) shows 2PEM images of gluten in the cleared noodle sample by SoROCS. (d) and (e) show images after protein network analysis with AngioTool (blue points: junction (branching points), red lines: gluten skeleton, yellow line: gluten outlines). An enlarged image of the section delineated with a red square is shown at the tip of the red arrow. Scale bars indicate 100 um. One of these eight different images obtained using CLSM and 2PEM is shown in (b) and (c), respectively.

[FIG. 2-3] Comparison of variables calculated by AngioTool for non-cleared noodles (blue) and cleared noodles (green): gluten percentage area (f), junction density (g), average gluten length (h), end points rate (i), and mean E lacunarity (j) are shown. Noodle samples were prepared four times independently (N = 4), and two different section samples were prepared from each noodle preparation (N = 2). Data f-j are presented as mean values ± SEM. The SEM is calculated from the mean value for N = 2. P values were calculated using the Student's two-tailed unpaired t test (f: P = 0.92, g: P = 0.76, h: P = 0.38, i: P = 0.31, j: P=0.76). All variables show no significant difference at the P = 0.05 level.

[FIG. 3-1] FIG. 3 shows two-photon excitation microscopy imaging of gluten. (a-e) show 2D gluten structure of noodle samples treated with SoROCS mixed with Thiolite™Green after fixation. The image processing was performed in the same manner for each set (a-e in FIG. 3-1, g-k in FIG. 3-3) to improve visibility. Scale bars indicate 200 μm. 2D images were excited at 920 nm (a), 930 nm (b), 940 nm (c), 950 nm (d), or 960 nm (e). This experiment was repeated three times using independently prepared samples, with results similar to those shown in (f) (FIG. 3-2).

[FIG. 3-2] Fluorescence intensity histograms in (f) were created using a 12-bit image obtained from a two-photon excitation microscopy (2PEM). The histograms are shown in (f) for 920 nm excitation (orange), 930 nm excitation (green), 940 nm excitation (red), 950 nm excitation (blue), and 960 nm excitation (purple), respectively. Fluorescence intensities up to ~1500 are shown.

[FIG. 3-3] (g-k) show 2D gluten structure of noodle samples treated with SoROCS mixed with AlexaFluor™ 633 $C_5$ Maleimide after fixation. Scale bars indicate 200 μm. 2D images were excited at 820 nm (g), 830 nm (h), 840 nm (i), 850 nm (j) or 860 nm (k). This experiment was repeated three times using independently prepared samples with results similar to those shown in (1) (FIG. 3-4).

[FIG. 3-4] Fluorescence intensity histograms (l) were created using a 12-bit images obtained from a two-photon excitation microscopy (2PEM). The histograms are shown in (l) for 820 nm excitation (orange), 830 nm excitation (green), 840 nm excitation (red), 850 nm excitation (blue), and 860 nm excitation (purple), respectively. Fluorescence intensities up to ~3000 are shown.

[FIG. 3-5] (m) is a merged image of gluten (magenta) and fluorescent microspheres (green) obtained using 2PEM. (n) is an enlarged image of the area marked with a red square in (m). Scale bars indicate 50 um (m) and 5 um (n), respectively. This experiment was repeated three times with independently prepared samples (the mean value and SD for FWHM were 0.54 and 0.03 um, respectively).

[FIG. 3-6] (o) shows a plot profile of fluorescence intensity (point spread function) for fluorescent microspheres along a red line shown in (n) (FIG. 3-5). Plots and lines shown in (o) indicate experimental data on the fluorescence intensity and calculated data using Gaussian function presented as equation (2) ($I_o$ = 156.2, $X_c$ = 5.16 pm, A =

1346.9, w = 0.509). Two black opposing arrows indicate FWHM. This experiment was repeated three times using independently prepared samples (the mean value and SD for FWHM were 0.54 and 0.03 um, respectively).

[FIG. 4-1] FIG. 4 shows 3D reconstruction of gluten in whole noodle. (a) shows experimental setup for 2PEM imaging.

[FIG. 4-2] (b) shows a comparison of depth imaging of noodles treated with water (left) and SoROCS (right). When rendering with FluoRender, gamma change (3.2) and alpha blending (500) were applied and a threshold was set to 146 and 90 for water and SoROCS, respectively.

[FIG. 4-3] (c) shows 3D reconstruction of gluten for noodle treated with water (left) and SoROCS (right). When rendering with FluoRender, gamma change (3.2) and alpha blending (500) were applied and a threshold was set to 146 and 90 for water and SoROCS, respectively.

[FIG. 4-4] (d) shows the fluorescent image quality of gluten depending on the depth from the surface of noodles. Data are presented as mean ± SD (N=3 independently prepared noodle samples). The 2D imaging conditions with 2PEM were adjusted to be optimal for each depth.

[FIG. 4-5] One of the section images obtained from three independently prepared noodle samples is shown in (e-h), respectively. The 2D imaging conditions with 2PEM were adjusted to be optimal for each depth. Optical sections were obtained at several depths: z = 550 um (e), Z = 1050 um (f), z = 1550 um (g) and Z = 2050 um (h). Scale bars indicate 100 um.

[FIG. 5-1] FIG. 5 shows 3D structure of gluten and stress-strain curves for various noodle samples. Skeletal structures of gluten labelled with Thiolite™ Green were imaged with 2PEM. 3D structures of gluten in control noodle (a), 10% gluten noodle (b) and 20% gluten noodle (c) are shown. (d), (f) and (h) are optical section images in (a-c), and (e), (g) and (f) are binarised images obtained by the Otsu method. The percentages of gluten calculated by the Otsu method for control noodle, 10% gluten noodle and 20% gluten noodle were 13.27 ± 1.01, 21.69 ± 1.02 and 29.60 ± 0.68, respectively (the values show mean ± SD for N = 3 independently prepared noodle samples). Scale bars indicate 100 um. When rendering with FluoRender, alpha blending (500) was applied (a-c).

[FIG. 5-2] Skeletal structures of gluten labelled with Thiolite™ Green were imaged with 2PEM. The 3D structure of gluten in 3% NaCl(j), 6% NaCl(k), 9% NaCl(l) and 12% NaCl(m) are shown. Scale bars indicate 100 um. When rendering with FluoRender, alpha blending (500) was applied (j-m).

[FIG. 5-3] The stress-strain curves are shown in (n) for control (black), 10% gluten (green), 20% gluten (orange), 3% NaCl (blue), 6% NaCl (yellow), 9% NaCl (purple) and 12% NaCl (red) . The strain was normalized by the thickness of each sample, and the stress value for every 0.1 strain from 0.1 to 0.9 was calculated by the interpolation complement method. Data are presented as mean ± SD (N = 20 different samples from the same noodle preparation).

[FIG. 6-1] FIG. 6 shows volumetric analysis of a 3D image. (a) shows 3D rendering in a 12% NaCl noodle. The 3D data set shown in this figure is the same as that in (m) in FIG. 5-2. (b) shows surface rendering by binarizing the fluorescence intensity of (a). Each gluten clump that is three-dimensionally isolated from others is displayed in a different color in (b). (c) is an image rendered from a different viewpoints of the 3D image shown in (b). Scale bars indicate 50 um (a,b) and 20 um (c).

[FIG. 6-2] (d) shows volume distribution of gluten shown in (b) of FIG. 6-1.

[FIG. 7-1] FIG. 7 shows 3D reconstruction of gluten in a noodle observed by confocal laser scanning microscopy. (a) shows experimental setup for confocal laser scanning microscopy (CLSM) imaging.

[FIG. 7-2] (b) shows 3D reconstruction of the gluten labelled with Thiolite™ Green. When rendering with FluoRender, gamma change (3.2), alpha blending (500) and threshold (90) were applied.

[FIG. 7-3] Optical sections at several depths: Z = 250 um (c) and Z = 550 um (d) are shown. Scale bars indicate 100 um. The 2D imaging conditions with CLSM were adjusted to be optimal for each depth.

[FIG. 7-4] (e) shows the fluorescent image quality of gluten depending on the depth from the surface of noodles. Data are presented as mean values ± SD (N = 3 independently prepared noodle samples). The 2D imaging conditions with CLSM were adjusted to be optimal for each depth. The images used to calculate the VAR values in (e) are shown in (c) and (d) of FIG. 7-3.

[FIG. 8] FIG. 8 shows effect of clearing reagents on sample fragility. Transparent images of noodles cleared with ClearSee (a) and SoROCS (b) are shown. The images of (a) and (b) are the same as in FIG. 1-1 (g) and (h), respectively. However, the image processing was performed in the same manner for both images to improve visibility. Scale bars indicate 5 mm. Red arrows indicate visible crevices.

[FIG. 9] FIG. 9 shows opacity measurements of noodle samples treated with various clearing reagents of the present disclosure. The values in the figure show mean ± SD for three independent experiments.

Embodiments to carry out the Invention

(Clearing reagent)

The clearing reagent of the present application comprises, as an active ingredient, a compound selected from

the group consisting of a benzenecarboxylic acid compound, a hydroxybenzene compound, a benzenesulfonic acid compound, a homosulfamine compound, and benzenesulfohydroxamic acid compound. The clearing reagent of the present application may comprise two or more compounds selected from the above group.

As used herein, the "benzenecarboxylic acid compound" includes benzenecarboxylic acid, and a salt of benzenecarboxylic acid with a monovalent or divalent metal atom (hereinafter also referred to as a benzenecarboxylic acid salt), and further includes their hydrates and hydrochlorides. The benzenecarboxylic acid refers to an aromatic compound containing one benzene ring wherein a hydrogen atom is substituted with a carboxyl group at the position of at least one carbon atom of six carbon atoms forming the benzene ring, and optionally having a substituent group at one or more other carbon atom positions. From the viewpoint of solubility, a salt of benzenecarboxylic acid or a hydrate thereof, or a hydrochloride of benzenecarboxylic acid is preferably used. The benzenecarboxylic acid salt may be a salt of one molecule of benzenecarboxylic acid with a monovalent metal atom, or a salt of one or two molecules of benzenecarboxylic acid with a divalent metal atom.

Examples of the substituent group that the benzene ring of the benzenecarboxylic acid compound may have include, but not limited to, a hydroxy group, an amino group, a nitro group, a carboxyl group, a sulfonic acid group, a sulfonamide group ($-S(O_2)NH_2$), an acetamide group ($-CH_2C(O)NH_2$), a hydrocarbon group, for example an alkyl group, an alkenyl group, or an alkynyl group etc., an alkoxy group, an alkoxycarbonyl group, an aldehyde group, and a halogen atom. The hydrocarbon group may be linear, branched or cyclic. The hydrocarbon group and the alkoxy group may be optionally substituted, for example, with one or more substituent groups selected from the group consisting of an amino group, an alkylamino group, a carbonyl group, an aminocarbonyl group, a hydroxy group, and a halogen atom.

The benzenecarboxylic acid compound may optionally have two or more carboxyl groups on the benzene ring, and has preferably one or two carboxyl groups on the benzene ring. When the benzenecarboxylic acid compound has two or more carboxyl groups on the benzene ring, at least one carboxyl group preferably forms a salt with a monovalent or divalent metal atom. For example, each of the two carboxyl groups may form a salt with a monovalent metal atom, or the two carboxyl groups may form a salt with a divalent metal atom.

As used herein, the "hydroxybenzene compound" refers to an aromatic compound containing one benzene ring wherein a hydrogen atom is substituted with a hydroxyl group at the position of at least one carbon atom of six carbon atoms forming the benzene ring, and optionally having a substituent group at one or more other carbon atom positions, and also includes a hydrate and a hydrochloride thereof.

Examples of the substituent group that the benzene ring of the hydroxybenzene compound may have include, but not limited to, a hydroxy group, an amino group, a nitro group, a sulfonic acid group, a sulfonamide group, an acetamide group, a hydrocarbon group, for example an alkyl group, an alkenyl group, and an alkynyl group etc., an alkoxy group, an alkoxycarbonyl group, an aldehyde group, and a halogen atom. The hydrocarbon group may be linear, branched or cyclic. The hydrocarbon group and the alkoxy group may be optionally substituted, for example, with one or more groups selected from the group consisting of an amino group, an alkylamino group, a carbonyl group, an aminocarbonyl group, a hydroxy group, and a halogen atom.

The hydroxybenzene compound may have two or more hydroxyl groups on the benzene ring, and has preferably one to three hydroxyl groups on the benzene ring.

As used herein, the "benzenesulfonic acid compound" includes benzenesulfonic acid, and a salt of benzenesulfonic acid with a monovalent or divalent metal atom (hereinafter also referred to as a benzenesulfonic acid salt), and further includes their hydrates and hydrochlorides. The benzenesulfonic acid refers to an aromatic compound containing one benzene ring wherein a hydrogen atom is substituted with a sulfonic acid group at the position of at least one carbon atom of six carbon atoms forming the benzene ring, and optionally having a substituent group at one or more other carbon atom positions. From the viewpoint of solubility, a salt of benzenesulfonic acid or a hydrate thereof, or a hydrochloride of benzenesulfonic acid is preferably used. The benzenesulfonic acid salt may be a salt of one molecule of benzenesulfonic acid with a monovalent metal atom, or a salt of one or two molecules of benzenesulfonic acid with a divalent metal atom. The benzenesulfonic acid is preferably a salt of one molecule of benzenesulfonic acid with a monovalent metal atom or a salt of two molecules of benzenesulfonic acid with a divalent metal atom.

Examples of the substituent group that the benzene ring of the benzenesulfonic acid compound may have include, but not limited to, a hydroxy group, an amino group, a nitro group, a sulfonic acid group, a sulfonamide group, an acetamide group, a hydrocarbon group, for example an alkyl group, an alkenyl group, and an alkynyl group etc., an alkoxy group, an alkoxycarbonyl group, an aldehyde group, a carboxyl group, and a halogen atom. The hydrocarbon group may be linear, branched or cyclic. The hydrocarbon group and the alkoxy group may be optionally substituted, for example, with one or more groups selected from the group consisting of an amino group, an alkylamino group, a carbonyl group, an aminocarbonyl group, a hydroxy group, and a halogen atom.

The benzenesulfonic acid compound may have two or more sulfonic acid groups on the benzene ring, and has preferably one or two sulfonic acid groups on the benzene ring. When the benzenesulfonic acid compound has two or more sulfonic acid groups on the benzene ring, at least one sulfonic acid group preferably forms a salt with a monovalent or divalent metal atom. For example, each of the two sulfonic acid groups may form a salt with a monovalent metal atom,

or the two sulfonic acid groups may form a salt with a divalent metal atom.

As used herein, the "homosulfamine compound" refers to an aromatic compound containing one benzene ring, having an aminomethyl group and an aminosulfonyl group at the para position on the benzene ring, and optionally having a substituent group at one or more other carbon positions, and also includes a hydrate and a hydrochloride thereof. Examples of the substituent group include, but not limited to, a hydroxy group, an amino group, a nitro group, a sulfonic acid group, a sulfonamide group, an acetamide group, a hydrocarbon group, for example an alkyl group, an alkenyl group, and an alkynyl group etc., an alkoxy group, an alkoxycarbonyl group, an aldehyde group, a carboxyl group, and a halogen atom. The hydrocarbon group may be linear, branched or cyclic. The hydrocarbon group and the alkoxy group may be optionally substituted, for example, with one or more groups selected from the group consisting of an amino group, an alkylamino group, a carbonyl group, an aminocarbonyl group, a hydroxy group, and a halogen atom. As the homosulfamine compound, for example, homosulfamine or a hydrate or hydrochloride thereof is used.

As used herein, the "benzenesulfohydroxamic acid compound" refers to an aromatic compound containing one benzene ring wherein a hydrogen atom is substituted with an N-hydroxysulfonamide group ($-S(O_2)NHOH$) at the position of at least one carbon atom of six carbon atoms forming the benzene ring, and optionally having a substituent group at one or more other carbon atom positions, and also includes a hydrate and a hydrochloride thereof. Examples of the substituent group include, but not limited to, a hydroxy group, an amino group, a nitro group, a sulfonic acid group, a sulfonamide group, an acetamide group, a hydrocarbon group, for example an alkyl group, an alkenyl group, and an alkynyl group etc., an alkoxy group, an alkoxycarbonyl group, an aldehyde group, a carboxyl group, and a halogen atom. The hydrocarbon group may be linear, branched or cyclic. The hydrocarbon group and the alkoxy group may be optionally substituted, for example, with one or more groups selected from the group consisting of an amino group, an alkylamino group, a carbonyl group, an aminocarbonyl group, a hydroxy group, and a halogen atom. As the benzenesulfohydroxamic acid compound, for example, benzenesulfohydroxamic acid is used.

Examples of the halogen atom include fluorine, chlorine, bromine, and iodine.

The monovalent metal atom may be, for example, an alkali metal atom. Examples of the alkali metal atom include lithium, sodium, potassium, rubidium and cesium, preferably lithium, sodium, potassium and cesium, more preferably lithium, sodium and potassium.

The divalent metal atom may be, for example, an alkaline earth metal atom, nickel, iron, or copper. Examples of the alkaline earth metal atom include calcium, strontium, barium, beryllium, and magnesium. Preferable examples of the divalent metal atom include calcium and nickel.

An example of the benzenecarboxylic acid compound is a compound represented by formula I:

[Chemical formula 4]

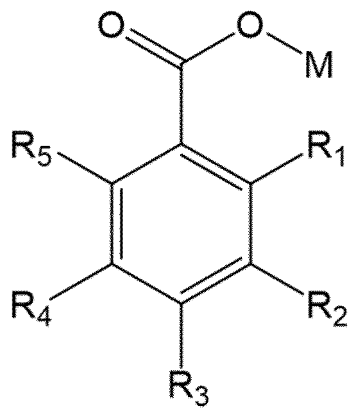

wherein,

- $R_1$ to $R_5$ independently represent a hydrogen atom, a hydroxy group, an amino group, a nitro group, a carboxyl group, a C1-C4 alkyl group, a C2-C4 alkenyl group, a C2-C4 alkynyl group, a C1-C4 alkoxy group, or a halogen atom, wherein the C1-C4 alkyl group, the C2-C4 alkenyl group, the C2-C4 alkynyl group, and the C1-C4 alkoxy group may be optionally substituted with a halogen atom, and
- M represents a monovalent or divalent metal atom or a hydrogen atom, and
- when any of $R_1$ to $R_5$ is a carboxyl group, the carboxyl group may form a salt together with a monovalent metal atom, or when $R_1$ or $R_5$ is a carboxyl group and M is a divalent metal atom, the carboxyl group and M may form a salt,

or a hydrate or hydrochloride thereof.

A further example of the benzenecarboxylic acid compound is a compound represented by Formula I, wherein one or two of $R_1$ to $R_5$ are hydroxy groups, or a hydrate or hydrochloride thereof.

A further example of the benzenecarboxylic acid compound is a compound represented by Formula I, wherein $R_1$ to $R_5$ are independently a hydrogen atom, a hydroxy group, an amino group, a nitro group, or a carboxyl group, or a hydrate or hydrochloride thereof.

A further example of the benzenecarboxylic acid compound is a compound represented by Formula I wherein M is a monovalent or divalent metal atom, or a hydrate or hydrochloride thereof. A further example of the benzenecarboxylic acid compound is a compound represented by Formula I wherein M is lithium, sodium, potassium or calcium, or a hydrate or hydrochloride thereof.

A further example of the benzenecarboxylic acid compound is a compound represented by Formula I wherein one or two of $R_1$ to $R_5$ are hydroxy groups and M is lithium, sodium, potassium or calcium, or a hydrate or hydrochloride thereof. A further example of the benzenecarboxylate compound is a compound represented by Formula I wherein $R_1$ to $R_5$ are independently a hydrogen atom, a hydroxy group, an amino group, a nitro group or a carboxyl group and M is lithium, sodium, potassium or calcium, or a hydrate or hydrochloride thereof.

Further examples of the benzenecarboxylic acid compound include, but not limited to, a salt of benzoic acid, hydroxybenzoic acid, aminobenzoic acid, nitrobenzoic acid, aminosalicylic acid, nitrosalicylic acid, or phthalic acid with a monovalent or divalent metal atom (hereinafter also referred to as benzoate, hydroxybenzoate, aminobenzoate, nitrobenzoate, aminosalicylate, nitrosalicylate, and phthalate, respectively), and a hydrate and a hydrochloride thereof. Further preferred examples of the benzenecarboxylic acid compound include, but not limited to, benzoate, 2-, 3-, or 4-hydroxybenzoate, and phthalate, and their hydrates.

Further, specific examples of the benzenecarboxylic acid compound include, but not limited to, sodium benzoate, potassium benzoate, sodium 2-hydroxybenzoate (sodium salicylate), lithium salicylate, sodium 3-hydroxybenzoate, 4-sodium hydroxybenzoate, sodium 2-aminobenzoate, sodium 3-aminobenzoate, sodium 4-aminobenzoate, potassium 4-nitrobenzoate, sodium 5-aminosalicylate, sodium 3-nitrosalicylate, disodium phthalate, and calcium phthalate.

An example of the hydroxybenzene compound is a compound represented by formula II:

[Chemical formula 5]

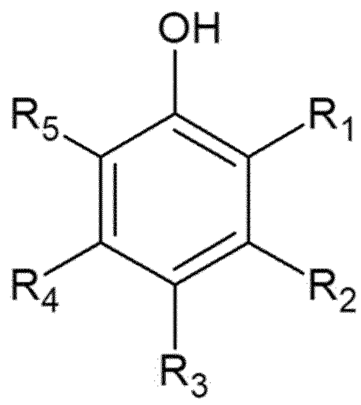

wherein,

$R_1$ to $R_5$ independently represent a hydrogen atom, a hydroxy group, an amino group, a nitro group, a sulfonic acid group, a sulfonamide group, an acetamide group, a 1-hydroxy-2-(methylamino)ethyl group, a methoxycarbonyl group, a C1-C4 alkyl group, a C2-C4 alkenyl group, a C2-C4 alkynyl group, a C1-C4 alkoxy group, or a halogen atom, wherein the C1-C4 alkyl group, the C2-C4 alkenyl group, the C2-C4 alkynyl group, and the C1-C4 alkoxy group may be optionally substituted with a halogen atom,

or a hydrate or hydrochloride thereof.

A further example of the hydroxybenzene compound is a compound represented by formula II wherein one or two of $R_1$ to $R_5$ are hydroxy groups, or a hydrate or hydrochloride thereof.

A further example of the hydroxybenzene compound is a compound represented by formula II wherein $R_1$ to $R_5$ are independently a hydrogen atom, a hydroxy group, an amino group, a sulfonamide group, an acetamide group, a 1-hydroxy-2-(methylamino)ethyl group, a methoxycarbonyl group or a halogen atom, or a hydrate or hydrochloride thereof.

Further examples of the hydroxybenzene compound include, but not limited to, catechol, resorcinol, pyrogallol, trihydroxybenzene, dichlororesorcinol, hydroxybenzenesulfonamide, hydroxyphenylacetamide, amino-hydroxybenzenesulfonic acid, methyl iodosalicylate, diaminoresorcinol, adrenaline, and their hydrates and hydrochlorides. Specific examples of the hydroxybenzene compound include, but not limited to, catechol, resorcinol, pyrogallol, 1,2,4-trihydroxybenzene, 4,6-dichlororesorcinol, 4-hydroxybenzenesulfonamide, 4-hydroxyphenylacetamide, 3-amino-4-hydroxybenzenesulfonic acid hydrate, methyl 4-iodosalicylate, 4,6-diaminoresorcinol dihydrochloride, and DL-adrenaline hydrochloride.

An example of the benzenesulfonic acid compound is a compound represented by formula III:

[Chemical formula 6]

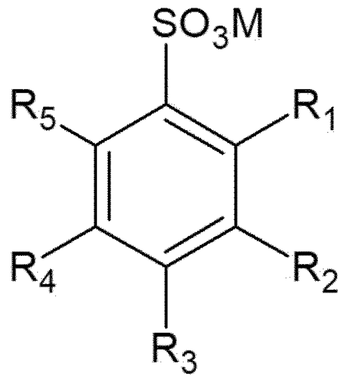

wherein,

$R_1$ to $R_5$ independently represent a hydrogen atom, a hydroxy group, a carboxyl group, an amino group, a nitro group, a sulfonic acid group, an aldehyde group, a C1-C4 alkyl group, a C2-C4 alkenyl group, a C2-C4 alkynyl group, a C1-C4 alkoxy group, or a halogen atom, wherein the C1-C4 alkyl group, the C2-C4 alkenyl group, the C2-C4 alkynyl group and the C1-C4 alkoxy group may be optionally substituted with a halogen atom, and
M represents a monovalent or divalent metal atom or a hydrogen atom, and
when any of $R_1$ to $R_5$ is a sulfonic acid group, the sulfonic acid group may form a salt together with a monovalent metal atom, or
when $R_1$ or $R_5$ is a sulfonic acid group and M is a divalent metal atom, the sulfonic acid group and M may form a salt,

or a hydrate or hydrochloride thereof.

A further example of the benzenesulfonic acid compound is a compound represented by formula III wherein one or two of $R_1$ to $R_5$ are hydroxy groups, or a hydrate or hydrochloride thereof.

A further example of the benzenesulfonic acid compound is a compound represented by formula III wherein $R_1$ to $R_5$ are independently a hydrogen atom, a hydroxy group, a carboxyl group, an amino group, a nitro group, a sulfonic acid group, an aldehyde group, a C1-C4 alkyl group (preferably C1-C3 alkyl group, more preferably C1-C2 alkyl group), a C1-C4 alkoxy group (preferably C1-C3 alkoxy group, more preferably C1-C2 alkoxy group), a halogen atom, or a C1-C4 alkyl group substituted with a halogen atom (preferably a C1-C3 alkyl group substituted with a halogen atom, more preferably a C1-C2 alkyl group substituted with a halogen atom, for example 2-bromoethyl or 1-bromoethyl), or a hydrate or hydrochloride thereof.

A further example of the benzenesulfonic acid compound is a compound represented by formula III wherein M is a monovalent or divalent metal atom, or a hydrate or hydrochloride thereof. A further example of the benzenesulfonic acid compound is a compound represented by formula III wherein M is lithium, sodium, potassium, calcium or nickel, or a hydrate or hydrochloride thereof.

A further example of the benzenesulfonic acid compound is a compound represented by formula III wherein one or two of $R_1$ to $R_5$ are hydroxy groups and M is lithium, sodium, potassium, calcium or nickel, or a hydrate or hydrochloride thereof. A further example of the benzenesulfonic acid compound is a compound represented by formula III wherein R to $R_5$ are independently a hydrogen atom, a hydroxy group, a carboxyl group, an amino group, a nitro group, a sulfonic acid group, an aldehyde group, a C1-C4 alkyl group (preferably C1-C3 alkyl group, more preferably C1-C2 alkyl group), a C1-C4 alkoxy group (preferably C1-C3 alkoxy group, more preferably C1-C2 alkoxy group), a halogen atom, or a C1-C4 alkyl group substituted with a halogen atom (preferably a C1-C3 alkyl group substituted with a halogen atom, more preferably a C1-C2 alkyl group substituted with a halogen atom, for example 2-bromoethyl or 1-bromoethyl) and M is lithium, sodium, potassium, calcium or nickel, or a hydrate or hydrochloride thereof.

Further examples of the benzenesulfonic acid compound include, but not limited to, benzenesulfonate, chlorobenzenesulfonate, iodobenzenesulfonate, hydroxybenzenesulfonate, nitrobenzenesulfonate, formylbenzenesulfonate, ethylbenzenesulfonate, bromoethyl-benzenesulfonate, benzenedisulfonate, dinitrobenzenesulfonate, formylbenzenedisulfonate, nitroaniline-sulfonate, amino-nitrobenzenesulfonate, aniline-disulfonate, toluidine-sulfonate, picrylsulfonate, dichloro-hydroxybenzenesulfonate, amino-methylbenzenesulfonate, benzenedisulfonate, hydroquinonesulfonate, guaiacolsulfonate, sulfobenzoic acid monosalt, sulfoisophthalic acid monosalt, tiron, and their hydrates and hydrochlorides.

Specific examples of the benzenesulfonic acid compound include, but not limited to, sodium benzenesulfonate, nickel (II) benzenesulfonate hexahydrate, sodium 4-chlorobenzenesulfonate, sodium 2-iodobenzenesulfonate, sodium 4-hydroxybenzenesulfonate, sodium 4-hydroxybenzenesulfonate dihydrate, sodium 3-nitrobenzenesulfonate, sodium 2-sulfobenzaldehyde, sodium 4-ethylbenzenesulfonate, sodium 4-(2-bromoethyl)benzenesulfonate, disodium 1,3-benzenedisulfonate, sodium 2,4-dinitrobenzenesulfonate, disodium 4-formylbenzene-1,3-disulfonate, sodium 2-nitroaniline-4-sulfonate, sodium 2-amino-5-nitrobenzenesulfonate, monosodium aniline-2,5-disulfonate, sodium o-toluidine-4-sulfonate tetrahydrate, sodium picrylsulfonate dihydrate, sodium 3,5-dichloro-2-hydroxybenzenesulfonate, nickel (II) 2-amino-5-methylbenzenesulfonate, potassium benzene-1,2-disulfonate, potassium hydroquinonesulfonate, potassium guaiacolsulfonate, monosodium 3-sulfobenzoate, monosodium 5-sulfoisophthalate, sodium 4-hydroxybenzenesulfonate dihydrate, and tiron monohydrate.

The clearing reagent of the present disclosure may be a compound itself selected from the group consisting of a benzenecarboxylic acid compound, a hydroxybenzene compound, a benzenesulfonic acid compound, a homosulfamine compound, and a benzenesulfohydroxamic acid compound, or may be a solution containing a compound selected from the group consisting of a benzenecarboxylic acid salt compound, a hydroxybenzene compound, a benzenesulfonic acid salt compound, a homosulfamine compound, and a benzenesulfohydroxamic acid compound.

When the clearing reagent of the present disclosure is in the form of a solution, the clearing reagent contains as a solvent, for example, water, a buffer, methanol, ethanol, propanol, ethyl acetate, isopropyl alcohol, or the like. The clearing reagent of the present disclosure comprises a hydroxybenzoic acid salt compound at a concentration of 0.5M or more, preferably 1M or more, more preferably 2M or more, further more preferably 3M or more, and even more preferably 4M or more. The higher the concentration is, the higher effect can be expected. However, considering the compound property, for example, it is believed that resorcinol and catechol cannot be probably dissolved at 9M or more. For example, the solubility of sodium salicylate in water at 20°C is up to about 4.125 M.

The compound selected from the group consisting of the benzenecarboxylic acid compound, the hydroxybenzene compound, the benzenesulfonic acid compound, the homosulfamine compound, and the benzenesulfohydroxamic acid compound can be dissolved in a solvent, for example, by stirring, heating or cooling, ultrasonication, or the like. If the compound is particularly difficult to dissolve, the compound may be added little by little to a solvent while being subjected to ultrasonication, heating, cooling, or the like, so that the compound may be dissolved up to a desired concentration.

The clearing reagent of the present disclosure may further contain a fluorescent dye. The fluorescent dye is not particularly limited. The fluorescent dye may be, for example, a commercially available fluorescent dye or a synthesized fluorescent dye. The fluorescent dye, as used herein, also includes a fluorescent label and a fluorescent microsphere. Fluorescent labeling is useful for visualizing carbohydrate or protein structure in a material using the clearing reagent of the present disclosure. The use of fluorescent labeling allows, for example, visualization of yeast and the like in a material and visualization of three-dimensional distribution inside a material. The fluorescent label is not particularly limited, and may be selected from fluorescent labels that target carbohydrate or protein of interest (for example, fluorescent substances that bind to the carbohydrate or protein). The fluorescent label may be commercially available or may be obtained by conjugating a fluorescent dye to a substance that specifically binds to a desired target (for example, an antibody or the like). Conjugation of a substance that specifically binds to a target and a fluorescent dye can be performed by a known method, and a person skilled in the art can appropriately prepare a desired fluorescent label. Examples of the fluorescent label and the fluorescent dye include, but not limited to, Thiolite™ Green (TG), Alexa Fluor™ 633 C5 Maleimide (AF), various Alexa Fluor™ dyes, fluorescein isothiocyanate, and rhodamine. The amount of the fluorescent dye contained in the clearing reagent of the present disclosure is not particularly limited, and may be appropriately determined by a person skilled in the art. Depending on the type of fluorescent dye used, for example, the clearing reagent of the present disclosure may contain about 0.0001 to 0.01% (wt/wt) of the fluorescent dye.

The clearing reagent of the present disclosure may further contain a surfactant. The surfactant is believed to be effective for delipidation of a material as well as increased permeability of the clearing agent to a cytoplasm and a cell wall. As the surfactant, a nonionic surfactant may be preferably used. Examples of the nonionic surfactant include, but not limited to, Triton X, Tween, and NP-40. The amount of the surfactant contained in the clearing reagent of the present disclosure is not particularly limited, and may be appropriately determined by a person skilled in the art. For example, the clearing reagent of the present disclosure may contain about 1 to 30% (wt/wt) of the surfactant.

The clearing reagent of the present disclosure may further contain a defatting agent. The defatting agent is

useful when the material contains lipids. Examples of the defatting agent include, but not limited to, 1,2-hexanediol, limonene, methanol, ethanol, propanol, hexane, ethyl acetate, and isopropyl alcohol. The amount of the defatting agent contained in the clearing reagent of the present disclosure is not particularly limited, and may be appropriately determined by a person skilled in the art. It was found for the first time in the present disclosure that limonene has defatting effect.

The clearing reagent of the present disclosure may further contain a decolorizing agent. The decolorizing agent is useful when the material is colored. Examples of the decolorizing agent include, but not limited to, 1,2-hexanediol, methanol, ethanol, propanol, hexane, ethyl acetate, and isopropyl alcohol. The amount of the decolorizing agent contained in the clearing reagent of the present disclosure is not particularly limited, and may be appropriately determined by a person skilled in the art. It was found for the first time in the present disclosure that 1,2-hexanediol has decolorizing effect.

The clearing reagent of the present disclosure may further contain glycerol, a carboxyvinyl polymer, hydroxypropyl methylcellulose, propylene glycol, macrogol, percoll, ficoll, polyethylene glycol, polyvinylpyrrolidone, sucrose, fructose, glucose, etc.

The clearing reagent of the present disclosure is capable of optically clearing carbohydrate- or protein-containing materials. The carbohydrate- or protein-containing material is not limited as long as it contains carbohydrate or protein, and may be any material, for example, derived from foods, biological bodies, or animals (fish, birds, amphibians, reptiles, mammals, etc.), plants, insects, or microorganisms. As used herein, the "food" includes food products, food materials, and processed food products. Examples of the food-derived carbohydrate or protein-containing material include, but not limited to, starch-containing food products, starch-containing food materials, wheat flour-based food materials, wheat flour-based food products, rice-based food materials, rice-processed food products, meat, processed meat food products, meat-containing food materials, fish, fish processed food products, fish-containing food materials, vegetables, fruits, and confectionery. Specific examples of the food-derived carbohydrate or protein-containing material include, but not limited to, noodles, fried noodles, bread, bread dough, cooked rice, meat, sausages and hams, and fish sausages.

The carbohydrate- or protein-containing material may or may not be pretreated, depending on the type of material. The pretreatment includes fixation, delipidation, decolorization, enzymatic treatment, adjustment of water content, and treatment with a fluorescent dye.

The fixation has, for example, an effect of preserving the material or stabilizing the structure of cells in the material. The fixation may be performed by a conventional method, for example, by immersing the material in a fixative such as formaldehyde, paraformaldehyde, glutaraldehyde, alcohol, dimethyl suberiminate, Bouin's fixative, or Carnoy's fixative. The fixed material may be embedded in an embedding agent such as OCT Compound or SCEM, and then frozen, or may be embedded in an embedding agent such as epoxy resin or paraffin.

If the carbohydrate- or protein-containing material contains lipids, the material may be preferably subjected to delipidation. The delipidation can be performed, for example, using a defatting agent. The delipidation may be carried out by a conventional method such as immersion. For example, the defatting agents as listed above can be used.

If the carbohydrate- or protein-containing material contains a pigment, like meat or fish meat including salmon, the material may be preferably subjected to decolorization. The decolorization can be performed, for example, using a decolorizing agent. The decolorization may be performed by a conventional method such as immersion. For example, the decolorizing agents as listed above can be used.

If the carbohydrate- or protein-containing material is a material that cracks when immersed in a liquid, like rice, it is preferable that the moisture content of the material is adjusted to about 22% wt/wt (dry weight) or more prior to immersion of the material in a solution containing the clearing reagent. The moisture content may be adjusted by a conventional method, for example, a gas phase adsorption method or the like.

If the carbohydrate- or protein-containing material has a cell wall, like rice, it is preferable that the material is subjected to enzymatic treatment to make microscopic holes in the cell wall. Examples of enzyme for such treatment include, but not limited to, cellulase, pectolyase, pectinase, and their mixtures. The temperature and time of the enzymatic treatment may be appropriately determined depending on the type and concentration of enzyme used. For example, the enzymatic treatment may be performed at room temperature to about 30°C for about 5 minutes to 2 hours.

The carbohydrate- or protein-containing material may also contain a fluorescent dye, either by treatment with a fluorescent dye in advance or by genetic engineering to express a fluorescent protein. The treatment with a fluorescent dye can be performed, for example, by mixing or injecting a fluorescent dye into the material. For example, when the material is a noodle or bread, the fluorescent dye may be previously dissolved in water to be added to wheat flour during manufacture of a noodle or bread. Examples of the fluorescent dye are as mentioned above.

Although the mechanism by which the clearing reagent of the present disclosure optically clears carbohydrate- or protein-containing materials is not fully understood, it is likely that, in the case of carbohydrate-containing materials such as starch, a hydroxybenzoate promotes gelatinisation of carbohydrates in the materials and thereby the materials become transparent. Gelatinisation is a phenomenon whereby carbohydrate particles such as starch begin to swell with a gradual increase in the transparency and viscosity when heated in the presence of water. Salt is one of substances that affect gelatinisation, and the order in which salt promotes gelatinisation follows the Hofmeister series. The order of the Hofmeister series of anions and cations is $OH^- > C_6H_4(OH)COO^- > SCN^- > I^- > Br^- > Cl^- > SO_4^{2-}$ and $Ba^{2+} > Ca^{2+}$

> $Mg^{2+}$ > $Li^+$ > $Na^+$ > $K^+$ > $Rb^+$ > $NH_4^+$. Hence, a hydroxybenzoate is a relatively strong gelatinisation promoter. Indeed, though sodium hydroxide was a candidate for further promotion of gelatinisation, sodium hydroxide was unsuitable for use as an optical clearing reagent due to its high pH made, i.e., because alkalies induce protein denaturation. Furthermore, three-dimensional imaging by optical sectioning requires fluorescent labelling of structures, whereas high pH solutions may induce fading fluorescence of labelling reagents.

In the case of protein-containing materials, it is likely that a hydroxybenzoate compound increases the refractive indices of the materials (to about 1.4 or higher) and thereby the materials become transparent. For example, as shown in Examples described below, the clearing reagent of the present disclosure containing sodium salicylate at concentration of 4M has a high refractive index of 1.455 at room temperature.

(Clearing method)

The clearing method of the present disclosure comprises treating the above-described carbohydrate- or protein-containing material with the clearing reagent. The clearing treatment may be performed by any method for impregnating the carbohydrate- or protein-containing material with the clearing reagent. For example, the material may be immersed in a solution containing the clearing reagent. When the clearing reagent is a solution, it may be used as it is. When the clearing reagent is a solid, the clearing reagent may be dissolved in the above-mentioned solvent at the above-mentioned concentration to prepare a solution. The temperature and time for immersion are not particularly limited and may be appropriately adjusted. Depending on the kind of material, for example, the material may be immersed in the clearing reagent at about 25°C to 30°C for about 1 hour to 4 days, preferably at about 10°C to 30°C for about 1 day to 6 weeks, more preferably at about 5°C to 30°C for about 3 days to 12 weeks.

Thus, a clarified carbohydrate- or protein-containing material is obtained. The cleared material comprises the clearing reagent, wherein the material is impregnated with the clearing reagent. The clearing process with the clearing reagent of the present disclosure is reversible. The material cleared by the clearing method of the present disclosure returns to its pre-treatment opaque state when the clearing reagent is removed from the material. For example, the cleared material can be returned to an opaque state by immersing the cleared material in water to dissolve the clearing reagent that has entered the material in the water.

As used herein, the term "clearing" means matching the refractive indices of the clearing reagent and the material, and means that the material treated with the clearing reagent has a high degree of light (especially visible light) transmittance as compared to the pre-treatment material and is in a state that something on the other side of the material can be seen through the material. Transparency can be evaluated, for example, by quantifying the opacity of a transmission image.

(Visualization method)

The visualization method of the present disclosure comprises optically clearing a carbohydrate- or protein-containing material by the clearing method of the present disclosure. The visualization method of the present disclosure may further comprise treating the material with a fluorescent dye.

If the carbohydrate- or protein-containing material has autofluorescence or the clearing reagent of the present disclosure used contains a fluorescent dye, the treatment with a fluorescent dye as described above is not essential. When the carbohydrate- or protein-containing material has autofluorescence or the clearing reagent of the present disclosure used contains a fluorescent dye, clearing the material results in visualization of carbohydrates or proteins in the material. For example, materials derived from plants such as wheat and rice can have autofluorescence. However, when the autofluorescence is weak, it is preferable that the plant-derived material is subjected to the treatment with a fluorescent dye.

According to the visualization method of the present disclosure, it is possible to observe a deep part of the material, for example, even at a depth of 1 mm to 2 mm from the surface of the material. According to the visualization method of the present disclosure, carbohydrates or proteins inside the material can be visualized, and their three-dimensional distribution can also be visualized. Furthermore, the presence and distribution of air bubbles in the material can be visualized based on a difference in refractive indices.

The treatment of the material with a fluorescent dye may be performed before, after, or simultaneously with the treatment of the material with the clearing reagent.

The material thus visualized can be observed with an optical microscope. The type of optical microscope is not particularly limited, and for example, a two-photon excitation microscope (2PEM), a confocal laser microscope (CLSM), a light sheet microscope, a structured illumination microscope, or the like can be used.

Furthermore, the structures of carbohydrates or proteins in the material can be visualized by using fluorescent labels for the carbohydrates or proteins in the material.

Furthermore, a microscopic image of the visualized material can be obtained by using appropriate computer

software, an imaging device such as a camera, a scanner, etc.

(Kit)

The kit for clearing a carbohydrate- or protein-containing material of the present disclosure comprises the above-described clearing reagent of the present disclosure. The kit for visualizing a carbohydrate- or protein-containing material of the present disclosure comprises the above-described clearing reagent of the present disclosure and a fluorescent dye, or the clearing reagent of the present disclosure further containing a fluorescent dye. The kit for clearing or visualizing a carbohydrate- or protein-containing material of the present disclosure may further comprise one or more selected from the group consisting of a fixative for fixation of the material, a defatting agent for delipidation of the material, and a decolorizing agent for decolorization of the material. When the clearing reagent of the present disclosure is a solid, the kit may further comprise a solvent for dissolving the clearing reagent.

(Device)

The device for clearing a carbohydrate- or protein-containing material of the present disclosure comprises the above-described clearing reagent of the present disclosure. The device for visualizing a carbohydrate- or protein-containing material of the present disclosure comprises the above-described clearing reagent of the present disclosure and a fluorescent dye, or the clearing reagent of the present disclosure further containing a fluorescent dye, and may further comprise an optical microscope. The device for visualizing a carbohydrate- or protein-containing material of the present disclosure may further comprise an imaging device. The optical microscope and imaging device are as described above. The device for clearing or visualizing a carbohydrate- or protein-containing material of the present disclosure may further comprise a container for the clearing treatment.

Hereinafter, the present invention is further explained in detail by reference to Examples which the present invention is not limited to.

EXAMPLES

(Materials and Methods)

Unless otherwise specified, reagents, samples (materials) and methods used in Examples are as follows.

Preparation of clearing reagent of the present disclosure

1. Preparation of SoROCS

Sodium salicylate (Nacalai tesque, 31821-45) was completely dissolved in Milli-Q water under stirring and heating at 70°C to prepare a 4M sodium salicylate solution. Then, Triton X-100 (Nacalai tesque, 35501-02) was added to the 4M sodium salicylate solution at a concentration of 0.1% (wt/wt) to prepare SoROCS. RI was measured at 30°C using a refractometer (Atago, PAL-BX/RI). SoROCS had a pH of 7.5 and a high RI of 1.455 at 30°C and was almost colorless.

For fluorescent labelling of gluten, Thiolite™ Green (TG) (AAT Bioquest, 21508) and Alexa Fluor™ 633 $C_5$ Maleimide (AF) (Invitrogen, A20342) dissolved in dimethyl sulfoxide (Wako Chemicals, 043-07216) at a concentration of 10 mM was added to the SoROCS to yield a final concentration of 0.0005% and 0.0002% (wt/wt), respectively.

2. Preparation of other clearing reagents

Each compound was completely dissolved in Milli-Q water under stirring and heating at 70°C to prepare a 4M sodium 3-hydroxybenzoate solution, a 3.5M sodium 4-hydroxybenzoate solution, a 3M sodium benzoate solution, a 4M lithium salicylate solution, a 3.5M disodium phthalate solution, a 4M catechol solution, a 4M resorcinol solution, and a 4M pyrogallol solution. When the compound was difficult to dissolve, the compound was dissolved little by little over time under ultrasonication or stirring to prepare a solution with a desired concentration.

Preparation of noodle samples

Noodles that do not contain excess gluten powder or sodium chloride (referred to as "control noodles") were prepared as follows. Mixed were 500 g of wheat flour for noodle making (Nisshin Seifun, crude protein 8.5%, ash 0.34%) and 160 g of Milli-Q water, followed by kneading using a mixer (Hobart, N50) for 20 minutes at 139 rpm to produce wheat

dough. The wheat dough was then put into a pasta-making machine (Bottene) equipped with a vacuum pump (Ulvac, DTC21) and extruded through an opening in a Teflon die with a width of 1.8 mm and a thickness of 0.7 mm (tagliolini No. 17) at 11.0-15.0 kPa (abs) to produce noodles.

Noodles containing excess gluten powder (Nacalai tesque, 17011-95) or sodium chloride (Nacalai tesque, 31320-05) were prepared as follows. Gluten powder was mixed with wheat flour at 10% and 20% by weight ratio before adding Milli-Q water. Wheat dough and then noodles were produced in the same manner as above. The noodles thus produced are referred to as "10% gluten" and "20% gluten", respectively. Sodium chloride was dissolved in Milli-Q water at weight ratios of 3, 6, 9 and 12% before mixing with wheat flour. Wheat dough and then noodles were produced in the same manner as above. The noodles thus produced are referred to as "3%-NaCl", "6%-NaCl", "9%-NaCl" and "12%-NaCl", respectively. The amount of Milli-Q water was changed depending on the amount of sodium chloride. The composition of each noodle sample is shown in Table 1.

[Table 1]

Table 1. Noodle sample composition

| | control noodle | 10%-gluten | 20%-gluten | 3%-NaCl | 6%-NaCl | 9%-NaCl | 12%-NaCl |
|---|---|---|---|---|---|---|---|
| wheat flour (g) | 500 | 450 | 400 | 500 | 500 | 500 | 500 |
| gluten powder (g) | 0 | 50 | 100 | 0 | 0 | 0 | 0 |
| water (g) | 160 | 160 | 160 | 169 | 178 | 187 | 196 |
| sodium chloride (g) | 0 | 0 | 0 | 15 | 30 | 45 | 60 |

Fixation and optical clearing

The samples were fixed in 4% (wt/wt) paraformaldehyde (PFA) in PBS at 30°C for 1.5 hours, washed twice in D-PBS, and immersed in ClearSee, SoROCS, or water (containing 0.5% (wt/wt) sodium azide to prevent spoilage) at 30°C with shaking for 3 days. ClearSee was prepared by mixing xylitol powder [Nacalai tesque, 36718-65; final concentration 10% (wt/vol)], sodium deoxycholate [Nacalai tesque, 10712-96; final concentration 15% (wt/vol)], and urea [Nacalai tesque, 35905-35; final concentration 25% (wt/vol)] in Milli-Q water.

Measurement of opacity

The opacity of the samples was determined using a cooled CCD camera system (GE Healthcare, ImageQuant LAS 4000). The sample was placed on the bottom of a glass dish with a midplane filled with ClearSee, SoROCS, or water (containing 0.5% (wt/wt) sodium azide to prevent spoilage) as a reference. Then, the sample was digitised by the CCD camera which was cooled to -25°C with a lens (F0.85 43 mm) using white transillumination. A gray value of 3,072 x 2,048-pixel 16-bit tiff image was obtained with an exposure time of 6 seconds and an iris of F2.8. The opacity of the sample was calculated by normalising the gray value of the sample with that of the background using free software (Fiji). The contrast C, which represents the difference in gray level, was calculated according to the following equation (1) from an image of the samples laid with a film on which a grid pattern was printed. The plot profile was measured using Fiji software.

Equation (1):

[Mathematical formula 1]

$$C = \frac{g_{\max} - g_{\min}}{g_{\max} + g_{\min}} \quad (1)$$

wherein $g_{max}$ and $g_{min}$ represent the maximum and minimum values of the gray level, respectively, in a 5-mm long plot profile.

Measurement of sample morphology

For the measurement of sample linear expansion, photographs of 5,472 x 3,648 pixels were taken of the top

of the samples placed on the bottom of a glass dish with a midplane filled with each clearing reagent by using a high-resolution digital camera (Canon, EOS 70D; ISO = 100, aperture value = 29, shutter speed = 0.5) with a lens (Canon, EFS 60 mm). Then, the aberration of image-forming capability (peripheral illumination, color blur and distortions) and the deterioration of the resolution resulting from diffraction phenomena in the photographs were corrected using software (Canon, Digital Photo Professional) based on the designed-lens value provided by the supplier. The 14-bit raw data were recorded in the 16-bit tiff format. Based on the images of sample width, linear expansion was determined using software (Fiji).

Two-photon excitation microscopy imaging

The samples were imaged using an upright or inverted two-photon excitation microscopy (2PEM) system (Olympus, upright: FV1200MPE-BX61WI, inverted: FV1000MPE-IX83) with a 25 x immersion objective lens (XLSLPLN25XGMP, a numerical aperture (NA) = 1.00, working distance (WD) = 8 mm, RI = 1.41-1.52, a correction collar) for SoROCS, a 25 x immersion objective lens (XLPlan N 25X, NA = 1.05, WD = 2 mm, a correction collar) for water, or a 10 x dry objective lens (UPLSAPO10X2, NA = 0.40, WD = 3.1 mm). The brightness compensation function in the z direction was used to change detector sensitivity and laser power. Fluorescence signals were quantified with a normal PMT for AF or GaAsP PMT for TG. Imaging was carried out with Fluoview FV10-ASW software Ver. 4.2c (Olympus) . TG and AF were excited at 940 nm and 840 nm, respectively, using InSight DeepSee (Spectra-Physics). Microscope settings including objective lenses and imaging conditions used in Examples and figures are summarised in Table 2-1 and Table 2-2.

[Table 2-1]

Table 2 Microscope settings and imaging conditions

| FIG. | | | Microscope settings | | | | | Imaging conditions | | | | |
|---|---|---|---|---|---|---|---|---|---|---|---|---|
| | | | Type | Objective lens | Immersion | Excita-tion (nm) | Laser power (%) | Filter (nm) | XY (pixel) | XY (um/pixcel) | Z (slice) | Z (um/slice) |
| 2 | b | CLSM | Inverted | UPLSAPO 20X | air | 473 | 1.4 | 490-590 | 1,024 | 0.621 | 1 | NA |
| | c | 2PEM | Inverted | UPLSAPO10X2 | air | 940 | 37 | 495-540 | 1,024 | 1.242 | 1 | NA |
| 3 | a | 2PEM | Inverted | UPLSAPO10X2 | air | 920 | 30 | 495-540 | 512 | 2.485 | 1 | NA |
| | b | 2PEM | Inverted | UPLSAPO10X2 | air | 930 | 30 | 495-540 | 512 | 2.485 | 1 | NA |
| | c | 2PEM | Inverted | UPLSAPO10X2 | air | 940 | 30 | 495-540 | 512 | 2.485 | 1 | NA |
| | d | 2PEM | Inverted | UPLSAPO10X2 | air | 950 | 30 | 495-540 | 512 | 2.485 | 1 | NA |
| | e | 2PEM | Inverted | UPLSAPO10X2 | air | 960 | 30 | 495-540 | 512 | 2.485 | 1 | NA |
| | g | 2PEM | Inverted | UPLSAPO10X2 | air | 820 | 30 | 647/57 | 512 | 2.485 | 1 | NA |
| | h | 2PEM | Inverted | UPLSAPO10X2 | air | 830 | 30 | 647/57 | 512 | 2.485 | 1 | NA |
| | i | 2PEM | Inverted | UPLSAPO10X2 | air | 840 | 30 | 647/57 | 512 | 2.485 | 1 | NA |
| | j | 2PEM | Inverted | UPLSAPO10X2 | air | 850 | 30 | 647/57 | 512 | 2.485 | 1 | NA |
| | k | 2PEM | Inverted | UPLSAPO10X2 | air | 860 | 30 | 647/57 | 512 | 2.485 | 1 | NA |
| | m | 2PEM | Upright | XLSLPlan N 25X | SoROCS | 940 | 9 | 520-560 | 1,600 | 0.212 | 1 | NA |
| | m | 2PEM | Upright | XLSLPlan N 25X | SoROCS | 840 | 9 | 647/57 | 1,600 | 0.212 | 1 | NA |

[Table 2-2]

| | | | | | | | | | | | | |
|---|---|---|---|---|---|---|---|---|---|---|---|---|
| 4 | b, c left | 2PEM | Upright | XLPLN 25X W | Water | 940 | 2.0–2.5 | 495-540 | 640 | 0.795 | 71 | 2.5 |
| | b, c right | 2PEM | Upright | XLSLPlan N 25X | SoROCS | 940 | 10–25 | 495-540 | 640 | 0.795 | 882 | 2.5 |
| | e | 2PEM | Upright | XLSLPlan N 25X | SoROCS | 940 | 11 | 495-540 | 640 | 0.795 | 1 | NA |
| | f | 2PEM | Upright | XLSLPlan N 25X | SoROCS | 940 | 20 | 495-540 | 640 | 0.795 | 1 | NA |
| | g | 2PEM | Upright | XLSLPlan N 25X | SoROCS | 940 | 55 | 495-540 | 640 | 0.795 | 1 | NA |
| | h | 2PEM | Upright | XLSLPlan N 25X | SoROCS | 940 | 66 | 495-540 | 640 | 0.795 | 1 | NA |
| 5 | a | 2PEM | Upright | XLSLPlan N 25X | SoROCS | 940 | 27 | 495-540 | 640 | 0.795 | 340 | 1.5 |
| | b | 2PEM | Upright | XLSLPlan N 25X | SoROCS | 940 | 24–31 | 495-540 | 640 | 0.795 | 340 | 1.5 |
| | c | 2PEM | Upright | XLSLPlan N 25X | SoROCS | 940 | 28–49 | 495-540 | 640 | 0.795 | 340 | 1.5 |
| | j | 2PEM | Upright | XLSLPlan N 25X | SoROCS | 940 | 30–38 | 495-540 | 640 | 0.795 | 340 | 1.5 |
| | k | 2PEM | Upright | XLSLPlan N 25X | SoROCS | 940 | 34–40 | 495-540 | 640 | 0.795 | 340 | 1.5 |
| | l | 2PEM | Upright | XLSLPlan N 25X | SoROCS | 940 | 30–36 | 495-540 | 640 | 0.795 | 340 | 1.5 |
| | m | 2PEM | Upright | XLSLPlan N 25X | SoROCS | 940 | 28–33 | 495-540 | 640 | 0.795 | 340 | 1.5 |
| 7 | b | CLSM | Inverted | UPLSAPO 20X | air | 473 | 5–50 | 490-590 | 640 | 0.994 | 311 | 2 |
| | c | CLSM | Inverted | UPLSAPO 20X | air | 473 | 23 | 490-590 | 640 | 0.994 | 1 | NA |
| | d | CLSM | Inverted | UPLSAPO 20X | air | 473 | 59 | 490-590 | 640 | 0.994 | 1 | NA |

Point spread function analysis

Fluorescent microspheres (yellow green fluorescent FluoSpheres, diameter = 0.49 μm, Invitrogen, F8813) were diluted with Milli-Q water to a concentration of 0.01% (vol/vol), and dispersed under ultrasoication for 15 minutes. Next, 160 g of water containing fluorescent microspheres was mixed with 500 g of wheat flour to prepare noodles in the same manner as described above. The noodles were fixed in 4% (wt/wt) PFA/PBS for 1.5 hours at 30°C, and immersed in AF-mixed SoROCS at 30°C with shaking for 3 days. Subsequently, 2PEM xy images of AF-labelled gluten and fluorescent microspheres at approximately 1 mm depth from the noodle surface were acquired at 840 nm and 940 nm excitation, respectively, as described above. The experimental data on the fluorescence intensity I of fluorescent microspheres (point-spread-function) was represented by the following Gaussian function (Equation (2)). Gaussian fitting was used to obtain the full width at half maximum (FWHM) values for x axes using OriginPro 8.1J SR3 software (OriginLab, Ver. 8.1.34.90) .
Equation (2):

[Mathematical formula 2]

$$I = I_0 + \frac{A \cdot \exp\{-4\ln(2)(x - x_c)^2 / w^2\}}{w\sqrt{\pi/4\ln(2)}}$$

where $I_0$ is the baseline fluorescence intensity, $x_c$ is the center position of the peak, A is the modulus, and w is the full width at half maximum (FWHM) of the peak.

Fluorescence image quality analysis

To examine the fluorescent image quality of gluten depending on the depth from the surface of noodles, the variance VAR was calculated from the histogram according to Equation (3). The 2D imaging conditions with 2PEM and CLSM were adjusted to be optimal for each depth, and the values of fluorescence intensity in blocked up shadows and blown out highlights were excluded when calculating the VAR values.
Equation (3):

[Mathematical formula 3]

$$VAR = \sum(I - I_p)^2 F(I)$$

wherein *I* is the fluorescence intensity, $I_p$ is the fluorescence intensity at the peak top of the histogram, and F(I) represents the normalised frequency for each fluorescence intensity I.

Confocal laser scanning microscopy Imaging

The samples were imaged using an inverted confocal laser scanning microscope (CLSM) system (Olympus, FV1000D IX81) with a 20 x dry objective lens (UPLSAPO 20X, NA = 0.75, WD = 0.6). The brightness compensation function in the z direction was used to change detector sensitivity and laser power. Imaging was carried out with Fluoview FV10-ASW software Ver. 3.0a (Olympus). TG and Alexa Fluor™ 594 were excited at 473 nm and 559 nm, respectively, using diode lasers. The imaging conditions for all figures are summarised in Table 2-1 and Table 2-2.

Protein network analysis

Software (National Cancer Institute, National Institute of Health, AngioTool64 Ver. 0.6a) was used to analyse the protein network characteristics for CLSM and 2PEM images of gluten skeletal structures. The same settings and parameters were applied to ensure reproducible quantification of the gluten network. Vessel thickness (as used herein, which corresponds to thickness of gluten skeletal structure) was set to 8, intensity low and high threshold were set to 0 and 255, small particles were removed under 30 pixels, and the function of fill holes was deactivated.

Immunohistochemistry staining

The samples were fixed in 4% (wt/wt) PFA/PBS for 1.5 hours at 30°C and washed twice in D-PBS. The samples were then embedded in OCT compound, sectioned at a thickness of 10 μm at -20°C using a cryostat (Leica, CM1860), and collected on slides. The OCT compound on the slide was then rinsed with distilled water. Subsequent immunohistochemistry staining was performed at room temperature. The slides were washed twice in D-PBS, blocked for 30 minutes using SuperBlock™ (PBS) blocking buffer (1:4 (D-PBS); Pierce Thermo scientific, 37515), and incubated for 1 hour with primary antibodies (1:1000 (D-PBS); anti-wheat gluten chicken-polyclonal IgY, Agrisera AB, AS09-571). The slides were then washed twice with D-PBS and incubated for 1 hour with a secondary antibody (1:200 (D-PBS); goat anti-chicken IgY with Alexa Fluor 594, Invitrogen, A11042). Next, the slides were washed twice in D-PBS and incubated for 30 minutes with TG (10 mM in DMSO) diluted with D-PBS at a final concentration of 0.05% (wt/wt). Fluorescent signals for TG and Alexa Fluor 594 were acquired at 473-nm and 559-nm excitation, respectively, using CLSM. The imaging conditions for all figures are summarized in Table 2-1 and Table 2-2.

Image reconstruction and re-slicing

Software (FluoRender) was used to visualise the 3D reconstructed image and obtain various 2D stacked images from an entire 3D data set for FIG. 4-2 (b), FIG.4-3 (c), FIG. 5-1 (a-c), FIG. 5-2 (j-m), and FIG. 7-2 (b). Imaris (registered trademark) x64 software (Bitplane, Ver. 8.3.1) was used to render the 3D data set and to analyse the volume distribution presented in FIG. 6-2.

Compression test

Mechanical property was characterised using a stress-strain curve, which showed the relationship between stress and strain during compression of the samples. The stress-strain curves were generated using a creep meter (Yamaden, RE2-33005B Rheoner II) equipped with a wedge-shaped plunger (No. 49) and a 20 N load cell at 0.1 mm/s. A sample cut to approximately 30 mm in length was placed on a cylindrical stand. Then, the force applied by the wedge-shaped plunger and the distance travelled were recorded in computer while the cylindrical stand rose. The strain normalised by the thickness of the sample was set to 99%.

Statistical analysis

Calculation of numerical data and statistical analysis were performed using Microsoft 365 Excel. Data are presented as mean ± SD or mean ± SEM as indicated. P values were calculated using the Student's two-tailed unpaired t test. $P < 0.05$ was considered statistically significant.

Example 1: Optical clearing of wheat noodles using the clearing reagent of the present disclosure 1

As the clearing reagent of the present disclosure, a 4M sodium salicylate aqueous solution was prepared, and 0.1% (wt/wt) of Triton X-100 as a surfactant was added to the solution. Specifically, sodium salicylate (Nacalai tesque, 31821-45) was completely dissolved in Milli-Q water under stirring and heating at 70°C, as described above in section "Materials and Methods". Triton X-100 (Nacalai tesque, 35501-02) was then added to a 4M sodium salicylate solution at a concentration of 0.1% (wt/wt). The solution (hereinafter referred to as "SoROCS") had a pH of 7.5 and a high RI of 1.455 at room temperature and was almost colorless.

Wheat noodle samples (control noodles) were prepared, fixed with 4% paraformaldehyde for 1.5 hours, and immersed in water (containing 0.5% (wt/wt) sodium azide to prevent spoilage), ClearSee, or SoROCS. ClearSee is a reagent that is able to make plant samples transparent as described in Non-patent Literature 1, and aims to remove autofluorescence of chlorophyll from green leaves. To evaluate the transparency of the noodle samples, the opacity of the transmitted light image was quantified.

When the noodle samples were fixed with 4% paraformaldehyde for 1.5 hours, the opacity increased from 3.54 ± 0.03 to 5.58 ± 0.05 (FIG. 1-2 (i)). After that, the noodles immersed in water remained opaque, whereas the noodles treated with ClearSee or SoROCS began to become transparent within 6 hours (FIG. 1-1 (a-h)). The opacity of the noodles treated with ClearSee or SoROCS for 3 days was 1.61 ± 0.04 and 1.27 ± 0.01, respectively (FIG. 1-2 (i)).

To further compare the effect of treatment with ClearSee and SoROCS on the transparency of noodles, a grid-printed film was placed under the noodles and examined how clearly the grid pattern penetrated the noodles. Compared to the noodles treated with ClearSee, the plot profile of the noodles treated with SoROCS demonstrated a large difference in grey levels between the line and the non-line sections of the grid, and the peaks of the grey level that reflect the lines were sharp (FIG. 1-3 (j-o)). The contrast C represents the difference in grey level and was calculated according to

equation (1). Plot profiles were measured using Fiji software. The resulting contrast C for ClearSee and SoROCS values in a 5-mm long plot profile (curly braces in FIG. 1-3(n-o)) was 0.10 and 0.31, respectively. Such high contrast indicates that SoROCS can further suppress light scattering, which is a common obstacle associated with deep imaging via optical sectioning. Of note, the noodles treated with ClearSee had visible crevices, implying that the noodle structure was destroyed (FIG. 8). A previous study reported that mouse brains became fragile and unwieldy when treated with a clearing reagent containing urea because urea caused partial denaturation and loss of cellular proteins. In contrast, the noodles treated with the clearing reagent of the present disclosure, SoROCS, which does not contain urea, did not show marked fragility (FIG. 8).

Example 2: Assessment of expansion by the clearing reagent of the present disclosure

Previously reported clearing reagents for biological samples caused large changes in sample volume. The present inventors investigated the morphological changes caused by the clearing reagent of the present disclosure. As a result, linear expansion was observed to increase for noodles immersed in water and SoROCS (FIG. 2-1(a)). Specifically, the linear expansion increased in the early stage, and subsequently gradually approached the maximum value. The linear expansion values following immersion in water and SoROCS for 3 days were 1.28 $\pm$ 0.01 and 2.23 $\pm$ 0.08, respectively. Taken together, these results suggest that the increase in opacity of the noodles immersed in water is due to the expansion of the noodles. In contrast, although the noodles immersed in SoROCS swelled considerably, their transparency was increased. This considerable expansion of SoROCS-treated samples may be due to the swelling of starch induced by sodium salicylate.

Next, to examine the effect of expansion on gluten structures, protein network analysis, which is an image analysis that allows for precise quantification of structural and morphological network attributes of the complex gluten structure, was conducted for non-cleared noodles and cleared noodles. First, noodle samples were fixed with 4% paraformaldehyde for 1.5 hours and mechanically sliced using a cryostat to capture a 2D image of gluten in the sample section (FIG. 2-2 (b)). Gluten was fluorescently labelled with Thiolite™ Green (TG), which reacts with thiol groups (cysteine residues) on proteins. Next, the noodle was cleared with TG-mixed SoROCS (the noodle was fixed with 4% paraformaldehyde for 1.5 hours before clearing), and the gluten was visualised by optical sectioning (FIG. 2-2 (c)). The mechanically sectioned images of non-cleared noodles (FIG. 2-2 (b)) and the optically sectioned images of cleared noodles (FIG. 2-2 (c)) were observed with 20x and 10x objective lenses, respectively, because the linear expansion of the noodles fixed with 4% paraformaldehyde for 1.5 hours and the noodles cleared with SoROCS for 3 days after fixation was 1.30 $\pm$ 0.01 and 2.23 $\pm$ 0.08, respectively (FIG. 2-1 (a)). Protein network analysis was conducted on both images using AngioTool (FIG. 2-2 (d, e)). AngioTool was originally developed for the quantitative analysis of angiogenesis. Considering that the gluten network shares many commonalities with blood vessels, AngioTool has been successfully applied for the accurate quantification of structural and morphological network attributes of gluten structures. By adjusting the parameters used in AngioTool with the skeletal structures of gluten (FIG. 2-2 (b-e)), variables that characterise the morphology of gluten structure: gluten percentage area, junction density, average gluten length, end points rate, and mean E lacunarity were obtained. No significant differences were observed in variables between the mechanical sectioning images of non-cleared noodles and the optical sectioning images of cleared noodles ($P > 0.05$ for each; FIG. 2-3 (f-j)). Although the expansion caused by the swelling of starch granules during the clearing process using SoROCS may be an artefact of deep imaging, nevertheless, it was found that expansion does not significantly impact the skeletal structures of gluten in terms of morphology.

Example 3: Fluorescence imaging of gluten using the clearing reagent of the present disclosure

To image with the 2PEM, the optimal wavelength for two-photon excitation was explored. Noodles cleared with TG-mixed or Alexa Fluor™ 633 $C_5$ Maleimide (AF)-mixed SoROCS were prepared, and the fluorescence intensity of the noodles was measured by changing the excitation wavelength within a range of 920 nm to 960 nm (FIG. 3-1 and FIG. 3-2 (a-f)) or a range of 820 nm to 860 nm (FIG. 3-3 and FIG. 3-4 (g-l)), respectively, at 10 nm intervals. The fluorescence intensity histogram of the obtained images showed a slightly higher count in the region of high fluorescence intensity between approximately 500 and 1,000 when excited at 940 nm for TG staining and between approximately 1,500 and 2,000 when excited at 840 nm for AF staining (FIG. 3-2 and FIG. 3-4 (f, l)).

Next, to examine the image resolution for SoROCS-treated samples obtained with 2PEM, noodles were kneaded with fluorescent microspheres (0.49 $\mu$m diameter), and subsequently fixed, and immersed in AF-mixed SoROCS. Fluorescent microspheres, located at a depth of approximately 1 mm from the surface of the noodles, excited by 940-nm laser, and fluorescently labelled gluten with AF excited at 840 nm were then imaged (FIG. 3-5 (m, n)). The experimental data on the fluorescence intensity I of fluorescent microspheres (point-spread-function) were well represented by the Gaussian function presented as equation (2). Since the obtained value of the full width at half maximum (FWHM) of the peak was similar to the diameter of the fluorescent microspheres, it was determined that SoROCS-treated samples can

be imaged with the resolution of 0.54 $\pm$ 0.03 $\mu$m (the value shows mean $\pm$ SD for FWHM obtained from three independently prepared noodle samples).

Having established clearing and imaging conditions, whole-noodle imaging was performed using TG-mixed SoROCS (FIG. 4-1 to FIG. 4-5). With the 2PEM, imaging at a depth over 2 mm was achieved (FIG. 4-2 and FIG.4-1 (b, c)), though SoROCS caused an approximately 2.23-fold linear expansion (FIG. 2-1 (a)). In contrast, the existing depth imaging in which a sample is not made to be transparent is limited to imaging at a depth up to approximately 50 $\mu$m (FIG. 4-2 and FIG. 4-3 (b, c)). Moreover, clearing with SoROCS made it possible to observe the entire structure of gluten formed around starch granules having a size of approximately 30 $\mu$m. Furthermore, to examine the fluorescent image quality of gluten depending on the depth from the surface of the noodles, the variance VAR was calculated from the histogram according to equation (3), which characterises the distribution of fluorescence intensity by level and indicates a smaller value when the fluorescence intensity is concentrated on a specific value. The VAR value became smaller with increasing depth from the surface of the noodles (FIG. 4-4 (d)). At a depth of 0.550 mm, the VAR value was 5.69 $\pm$ 0.31 $\times$ $10^5$. When the depth exceeded 2 mm, the VAR value decreased significantly to 8.98 $\pm$ 3.81 $\times$ $10^4$. However, the skeletal structure of gluten was confirmed even at a depth over 2 mm (FIG. 4-4 and FIG. 4-5 (d-h)). To the best of our knowledge, this is the first report that the 3D structure of gluten was rendered, especially at millimetre-scale size and submicron resolution.

Example 4: Observation of dramatic changes in gluten structure by the clearing reagent of the present disclosure

Since use of SoROCS allowed successful imaging of a whole noodle, this Example was performed to demonstrate imaging of other gluten skeletal structures. Noodles were prepared by adding gluten powder or sodium chloride to wheat flour. Adding gluten powder to flour at 10% or 20% weight ratio aimed to create an additional auxiliary structure between the gluten skeleton formed in the wheat dough. The noodles thus produced are referred to as 10%- or 20%-gluten noodle, respectively. As expected, compared to the noodles without any addition (referred to as control noodle), the addition of gluten powder did not change the main skeletal structure composed of gluten, tough the overall structure became hazy (FIG. 5-1 (a-c)). To illustrate this, an optical section image was binarised to calculate the percentage of gluten in the image. As a result, it was found that the percentages of gluten for the control noodle, 10%-gluten noodle and 20%-gluten noodle were 13.27 $\pm$ 1.01%, 21.69 $\pm$ 1.02% and 29.60 $\pm$ 0.68% (the value shows mean $\pm$ SD for N = 3 independently prepared noodle samples), respectively. These results indicate that increased amounts of gluten powder caused an increase in the formation of gluten networks (FIG. 5-1 (d-i)). In contrast, the addition of sodium chloride at weight ratios of 3%, 6%, 9% and 12% was intended to alter the skeletal structures of gluten, and the noodles thus produced are referred to as 3%-NaCl, 6%-NaCl, 9%-NaCl and 12%-NaCl noodles, respectively. While there are several possible explanations for the mechanism by which sodium chloride alters the skeletal structure of gluten, sodium chloride likely shields charges on the gluten, thus limiting electrostatic repulsion between gluten polymers and causing them to aggregate. As the sodium chloride concentration increased to 3% and 6%, the structure of gluten dramatically changed from a relatively uniform honeycomb-shaped network to a sparse large clump (FIG. 5-2 (j, k)). Subsequently, when the sodium chloride concentration was further increased to 9% and 12%, the aggregated large clumps were dispersed, though these concentrations were not realistic (FIG. 5-2 (l, m)).

Next, to examine the effect of the changes in the skeletal structures on the mechanical properties, a compression test was performed (FIG. 5-3 (n)). For the 10%-and 20%-gluten noodles, the stress strain curve obtained when compressing the noodles with a wedge-shaped plunger shifted to the upper side. Conversely, the stress strain curves of the noodles containing sodium chloride moved downward. These results suggest that reinforcement of the gluten skeletal structure increases structural strength, whereas cleavage of the gluten network decreases structural strength. Notably, the dramatic change in the main skeletal structure of gluten had a great impact on the mechanical properties of wheat dough. These results are consistent with previous research on the physicochemical and structural changes of gluten. The high-resolution 3D reconstruction of SoROCS may facilitate structural insight into the mechanical properties of wheat dough.

As a further demonstration of this imaging methodology with SoROCS, gluten volume was quantified employing 3D measurement. First, the fluorescence intensity was binarised with respect to the 3D image of gluten in the 12%-NaCl noodles (FIG. 6-1 (a, b)). Each gluten clump that is three dimensionally isolated from the others is represented by a different colour in FIG. 6-1 (b). Notably, the positional relationship of each gluten clump can be readily observed from any angle and direction (FIG. 6-1 (c)). Next, when the volume distribution of gluten was analysed, a small number of huge gluten clumps were found to occupy most of the total product (FIG. 6-2 (d)). Information on the volume distribution that can be obtained only by this imaging methodology with SoROCS will serve to improve the industrial performance of wheat flour foods.

Example 5: Imaging using confocal laser scanning microscopy

Although 2PEM is a powerful tool for deep imaging, it is not accessible to all researchers due to its high equipment cost. Thus, confocal laser scanning microscopy (CLSM) observation of SoROCS-treated noodles was evaluated regarding imaging performance. Using CLSM, cleared noodle images were obtained (FIG. 7-2 (a, b)). The xy image at a depth of 250 μm from the noodle surface depicts clear gluten structures (FIG. 7-3(c)). To examine the fluorescent image quality of gluten, the VAR was calculated from the histogram for xy images at several depths (FIG. 7-3 and FIG. 7-4 (c-e)). The VAR value tended to decrease as the depth from the surface of the noodles increased, though there was no significant difference observed (FIG. 7-4 (e)). The working distance (WD) of the objective lens used in this Example limits the imaging depth to 600 μm. However, given that the gluten surrounding starch granules of about 30 μm in size forms a honeycomb-shaped network, SoROCS is compatible with imaging using CLSM as well as 2PEM.

Example 6: Optical clearing of wheat noodles using the clearing reagent of the present disclosure 2

In the same manner as in Example 1, wheat noodle samples were treated with, as the clearing reagent, a 4M sodium 2-hydroxybenzoate (sodium salicylate) solution (SoROCS), a 4M sodium 3-hydroxybenzoate solution, a 3.5M sodium 4-hydroxybenzoate solution, a 3M sodium benzoate solution, a 4M lithium salicylate solution, a 3.5M disodium phthalate solution, a 4M catechol solution, a 4M resorcinol solution, and a 4M pyrogallol solution, and optical clearing was assessed. The noodle samples were fixed and then immersed in each clearing reagent at 30°C for 3 days, and the opacity was measured. As controls, the opacity of the noodle sample just after fixation and the noodle sample immersed in D-PBS instead of the clearing reagent was measured. As a result, the samples were cleared when treated with any of the clearing reagents. The measurement results of opacity are shown in FIG. 9. Values in the figure represent mean ± SD of three independent experiments.

Example 7: Optical clearing of various samples using the clearing reagent of the present disclosure

A 4M sodium salicylate solution (SoROCS) was used as the clearing reagent to perform clearing experiments of various samples. Rice, pork, fish (salmon and sea bream), and vegetables (radish sprouts) were used as samples. Each sample was appropriately pretreated and then subjected to a clearing treatment.

The pretreatment for rice comprised adjustment of the moisture content to about 22% wt/wt (dry weight) or more by vapor phase adsorption, fixation with 4% PFA/PBS, and then enzymatic treatment with 1 wt% cellulase and 0.5 wt% pectolyase. Pork, fish (salmon and sea bream), and vegetables (radish sprouts) were cut into appropriate sizes, fixed in 4% PFA/PBS, and then subjected to delipidation and decolorization. Each sample pretreated as described above was immersed in the clearing reagent. As a result, all of the samples became transparent.

Example 8: Test for decolorizing agent and defatting agent

Pasta samples made from durum wheat were fixed in 4% (wt/wt) PFA/PBS at 30°C for 1.5 hours, and washed twice in D-PBS. The samples were immersed in 1,2-hexanediol or water as a control for 5 days, and then immersed in a 4M sodium 2-hydroxybenzoate (sodium salicylate) solution (SoROCS) for 3 days. As a result, carotenoid pigments in durum wheat were removed by the treatment with 1,2-hexanediol and thereby significant clearing was achieved with an opacity of 1.041 ± 0.002, whereas the opacity of the control pasta sample treated with water was 1.079 ± 0.002. Values represent mean ± SD of three independent experiments.

Wheat noodle samples fried in vegetable oil were fixed in 4% (wt/wt) PFA/PBS at 30°C for 1.5 hours and washed twice in D-PBS. The samples were immersed in limonene or water as a control for 5 days, and then immersed in a 4M sodium 2-hydroxybenzoate (sodium salicylate) solution (SoROCS) for 3 days. As a result, the oil in the fried noodle sample was removed by the treatment with limonene and thereby significant clearing was achieved with an opacity of 1.089 ± 0.004, whereas the opacity of the control pasta sample treated with water was 1.120 ± 0.006. Values represent mean ± SD of three independent experiments.

**Claims**

1. A reagent for optically clearing a carbohydrate- or protein-containing material, comprising a compound selected from the group consisting of a benzenecarboxylic acid compound, a hydroxybenzene compound, a benzene sulfonic acid compound, a homosulfamine compound, and a benzenesulfohydroxamic acid compound.

2. The reagent according to claim 1, wherein the reagent comprises a benzenecarboxylic acid compound, and the

benzenecarboxylic acid compound is a compound represented by formula I:

[Chemical formula 1]

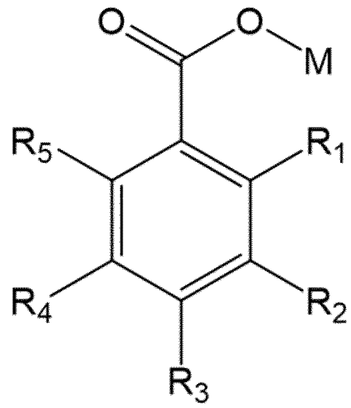

wherein,

$R_1$ to $R_5$ independently represent a hydrogen atom, a hydroxy group, an amino group, a nitro group, a carboxyl group, a C1-C4 alkyl group, a C2-C4 alkenyl group, a C2-C4 alkynyl group, a C1-C4 alkoxy group, or a halogen atom, wherein the C1-C4 alkyl group, the C2-C4 alkenyl group, the C2-C4 alkynyl group, and the C1-C4 alkoxy group may be optionally substituted with a halogen atom, and
M represents a monovalent or divalent metal atom or a hydrogen atom, and
when any of $R_1$ to $R_5$ is a carboxyl group, the carboxyl group may optionally form a salt together with a monovalent metal atom, or
when $R_1$ or $R_5$ is a carboxyl group and M is a divalent metal atom, the carboxyl group and M may optionally form a salt,

or a hydrate or hydrochloride thereof.

**3.** The reagent according to claim 2, wherein one or two of $R_1$ to $R_5$ represent a hydroxy group.

**4.** The reagent according to claim 2, wherein the benzenecarboxylic acid compound is a salt of benzoic acid, 2-, 3- or 4-hydroxybenzoic acid, or phthalic acid with a monovalent or divalent metal atom, or a hydrate thereof.

**5.** The reagent according to any one of claims 2 to 4, wherein M is lithium, sodium, potassium, or calcium.

**6.** The reagent according to claim 1, wherein the reagent comprises a hydroxybenzene compound, and the hydroxybenzene compound is a compound represented by formula II:

[Chemical formula 2]

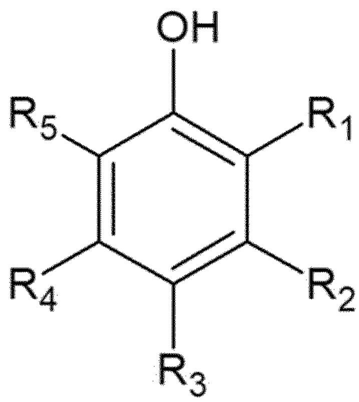

wherein,
$R_1$ to $R_5$ independently represent a hydrogen atom, a hydroxy group, an amino group, a nitro group, a sulfonic acid group, a sulfonamide group, an acetamide group, a 1-hydroxy-2-(methylamino)ethyl group, a methoxycarbonyl group, a C1-C4 alkyl group, a C2-C4 alkenyl group, a C2-C4 alkynyl group, a C1-C4 alkoxy group, or a halogen atom, wherein the C1-C4 alkyl group, the C2-C4 alkenyl group, the C2-C4 alkynyl group, and the C1-C4 alkoxy group may be optionally substituted with a halogen atom,
or a hydrate or hydrochloride thereof.

**7.** The reagent according to claim 6, wherein one or two of $R_1$ to $R_5$ represent a hydroxy group.

**8.** The reagent according to claim 6, wherein the hydroxybenzene compound is catechol, resorcinol, or pyrogallol, or a hydrate thereof.

**9.** The reagent according to claim 1, wherein the reagent comprises a benzenesulfonic acid compound, and the benzenesulfonic acid compound is represented by formula III:

[Chemical formula 3]

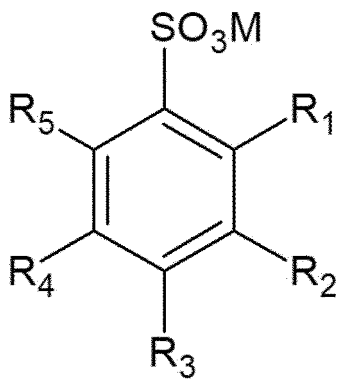

wherein,

$R_1$ to $R_5$ independently represent a hydrogen atom, a hydroxy group, a carboxyl group, an amino group, a nitro group, a sulfonic acid group, an aldehyde group, a C1-C4 alkyl group, a C2-C4 alkenyl group, a C2-C4 alkynyl group, a C1-C4 alkoxy group, or a halogen atom, wherein the C1-C4 alkyl group, the C2-C4 alkenyl group, the C2-C4 alkynyl group and the C1-C4 alkoxy group may be optionally substituted with a halogen atom, and
M represents a monovalent or divalent metal atom or a hydrogen atom, and
when any of $R_1$ to $R_5$ is a sulfonic acid group, the sulfonic acid group may optionally form a salt together with a monovalent metal atom, or
when $R_1$ or $R_5$ is a sulfonic acid group and M is a divalent metal atom, the sulfonic acid group and M may optionally form a salt,

or a hydrate or hydrochloride thereof.

**10.** The reagent according to claim 9, wherein one or two of $R_1$ to $R_5$ represent a hydroxy group.

**11.** The reagent according to claim 9 or 10, wherein M is lithium, sodium, potassium, calcium, or nickel.

**12.** The reagent according to any one of claims 1 to 11, further comprising a fluorescent dye.

**13.** A method of optically clearing a carbohydrate- or protein-containing material, the method comprising treating the carbohydrate- or protein-containing material with the reagent according to any one of claims 1 to 12.

**14.** The method according to claim 13, which comprises treating the material with the reagent after defatting and/or decolorizing the material by pretreatment.

**15.** The method according to claim 14, wherein the material is subjected to pretreatment with a decolorizing agent comprising 1,2-hexanediol and/or a defatting agent comprising limonene.

**16.** A method of visualizing carbohydrates or proteins in a carbohydrate-containing material, the method comprising optically clearing the material by the method according to any one of claims 13 to 15.

**17.** The method according to claim 16, further comprising treating the material with a fluorescent dye.

**18.** The method according to claim 16 or 17, further comprising imaging.

**19.** The method according to any one of claims 16 to 18, wherein the material is a wheat flour-based food material or a wheat flour processed food, and gluten protein is visualized.

**20.** A kit for optically clearing a carbohydrate- or protein-containing material, comprising the reagent according to any one of claims 1 to 12.

**21.** The kit according to claim 20, further comprising one or more selected from the group consisting of a fixative, a defatting agent, and a decolorizing agent.

**22.** The kit according to claim 21, which comprises a decolorizing agent comprising 1,2-hexanediol and/or a defatting agent comprising limonene.

**23.** A kit for visualizing carbohydrates or proteins in a material, comprising:

(a) the reagent according to any one of claims 1 to 11, and a fluorescent dye, or
(b) the reagent according to claim 12.

**24.** The kit according to claim 23, further comprising one or more selected from the group consisting of a fixative, a defatting agent, and a decolorizing agent.

**25.** The kit according to claim 24, which comprises a decolorizing agent comprising 1,2-hexanediol and/or a defatting agent comprising limonene.

**26.** A device for visualizing carbohydrates or proteins in a material, comprising:

(a) the reagent according to any one of claims 1 to 11, a fluorescent dye a reagent, and an optical microscope, or
(b) the reagent according to claim 12, and an optical microscope.

**27.** A cleared, carbohydrate- or protein-containing material, comprising the reagent according to any one of claims 1 to 12.

**28.** A decolorizing agent containing 1,2-hexanediol.

**29.** A defatting agent containing limonene.

[FIG. 1-1]

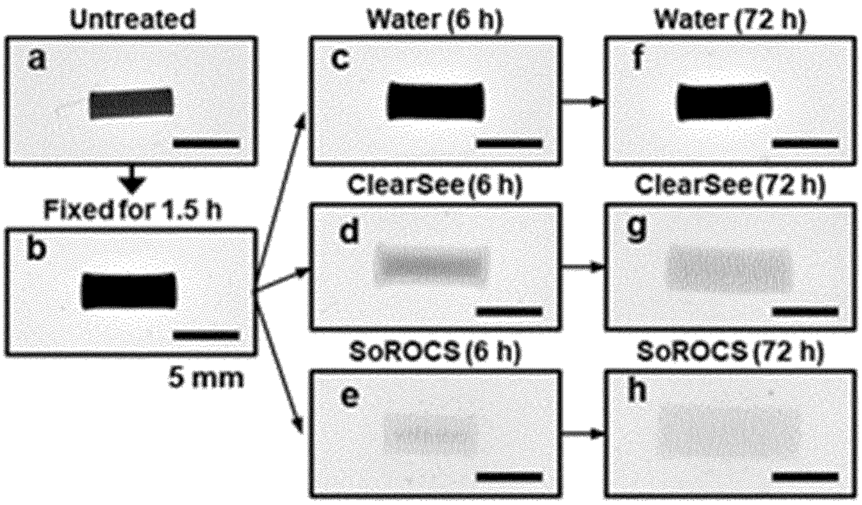
Untreated
a
Fixed for 1.5 h
b
5 mm
Water (6 h)
c
ClearSee (6 h)
d
SoROCS (6 h)
e
Water (72 h)
f
ClearSee (72 h)
g
SoROCS (72 h)
h

[FIG. 1-2]

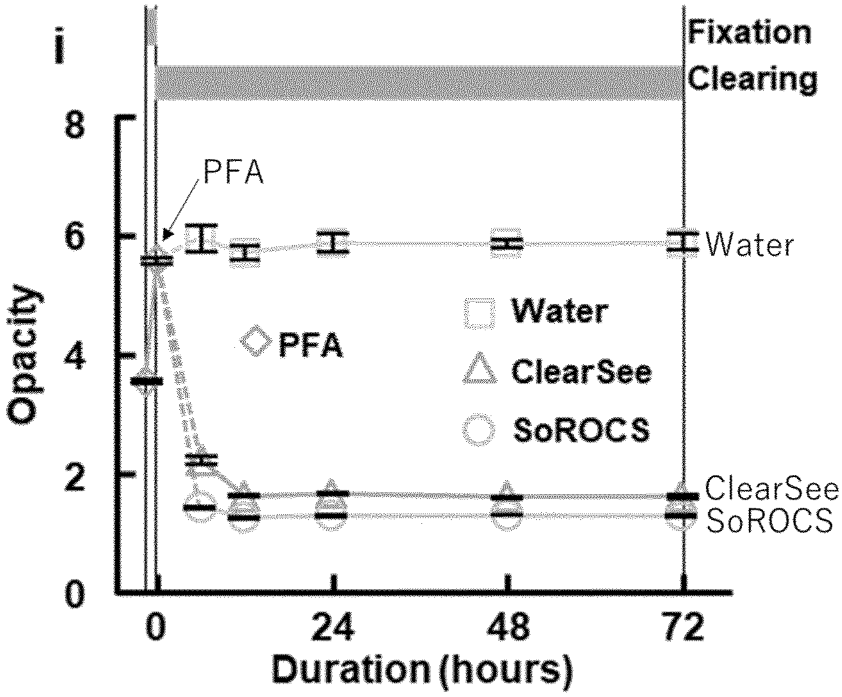
i
Fixation
Clearing
PFA
Water
PFA
Water
ClearSee
SoROCS
ClearSee
SoROCS
Opacity
0
2
4
6
8
0
24
48
72
Duration (hours)

[FIG. 1-3]

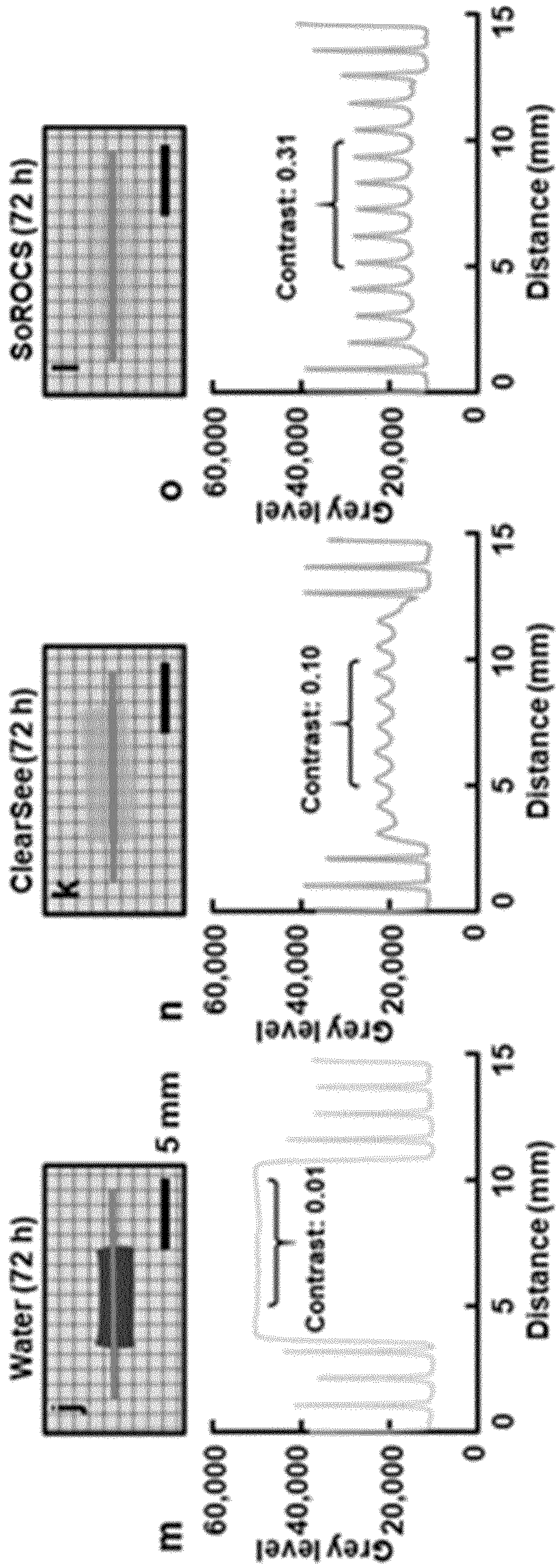
Water (72 h)
ClearSee (72 h)
SoROCS (72 h)
j
k
l
5 mm
m
n
o
Grey level
60,000
40,000
20,000
0
Distance (mm)
0
5
10
15
Contrast: 0.01
Contrast: 0.10
Contrast: 0.31

[FIG. 2-1]

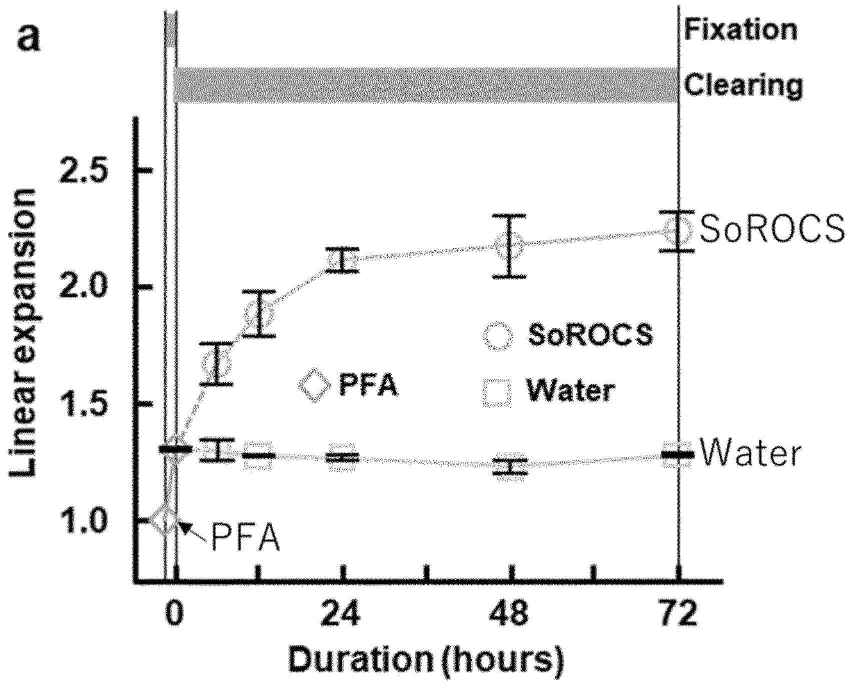
a
Fixation
Clearing
Linear expansion
2.5
2.0
1.5
1.0
SoROCS
PFA
Water
0
24
48
72
Duration (hours)

[FIG. 2-2]

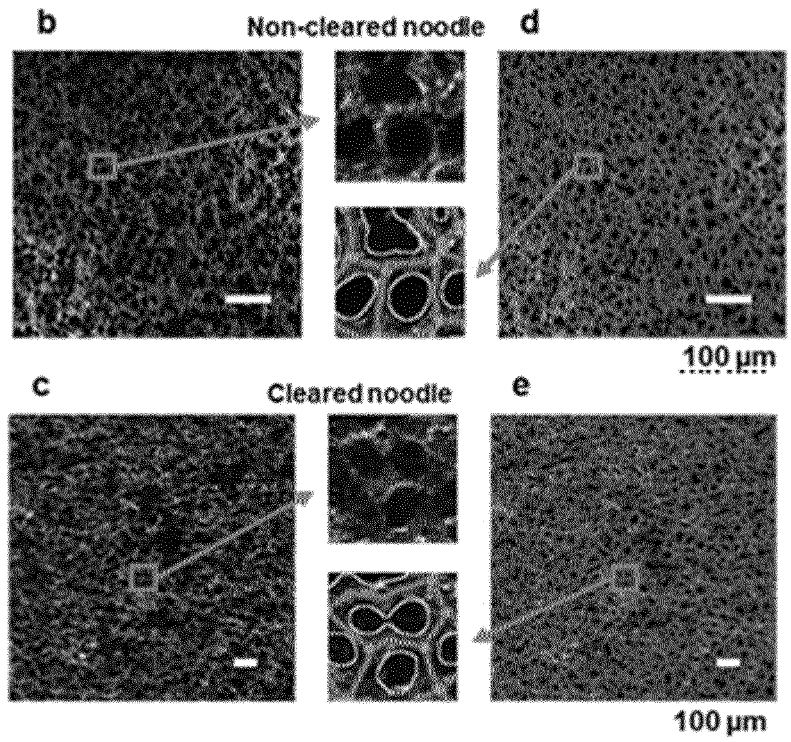
b
Non-cleared noodle
d
100 µm
c
Cleared noodle
e
100 µm

[FIG. 2-3]

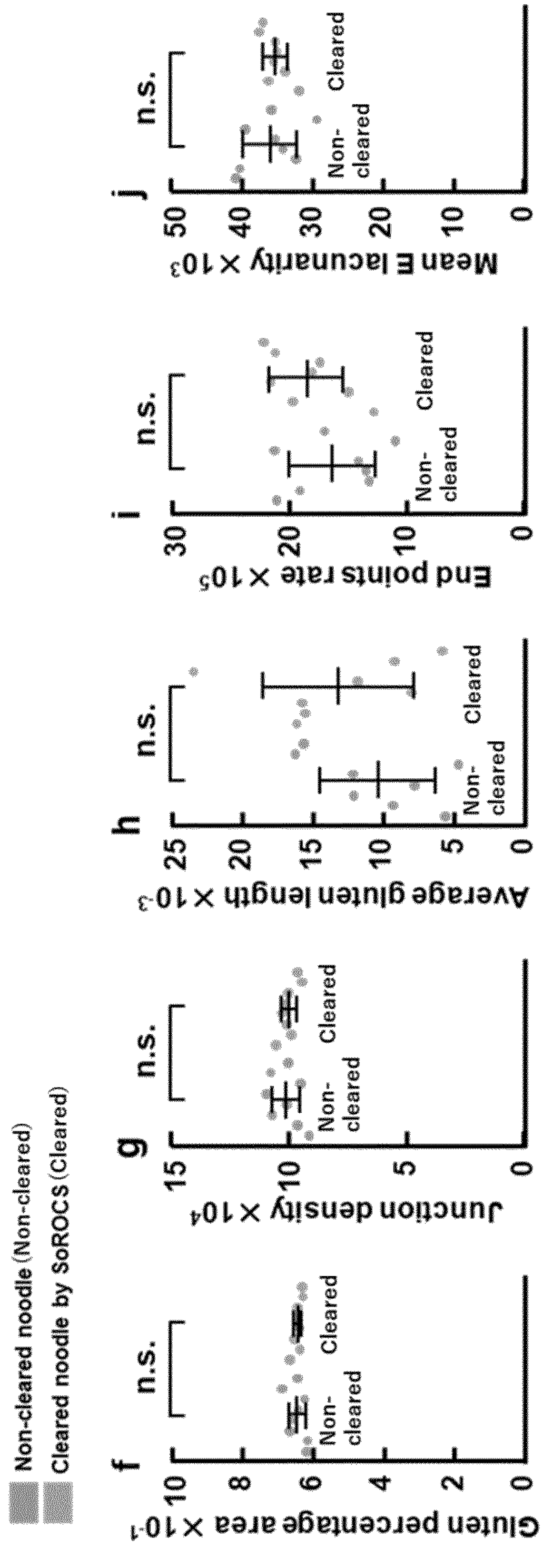
Non-cleared noodle (Non-cleared)
Cleared noodle by SoROCS (Cleared)
f
Gluten percentage area ×10-1
n.s.
Non-cleared
Cleared
g
Junction density ×104
n.s.
Non-cleared
Cleared
h
Average gluten length ×10-3
n.s.
Non-cleared
Cleared
i
End points rate ×105
n.s.
Non-cleared
Cleared
j
Mean E lacunarity ×103
n.s.
Non-cleared
Cleared

[FIG. 3-1]

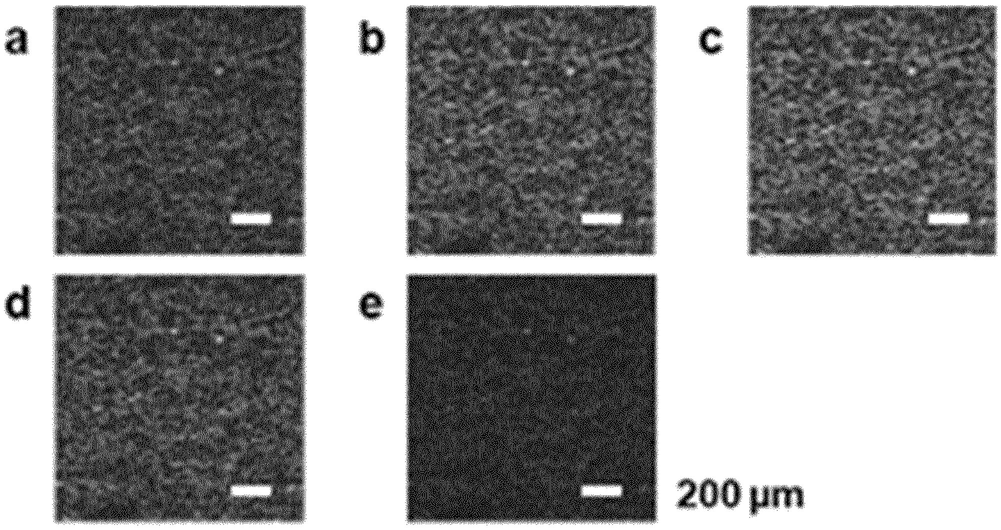
a
b
c
d
e
200 μm

[FIG. 3-2]

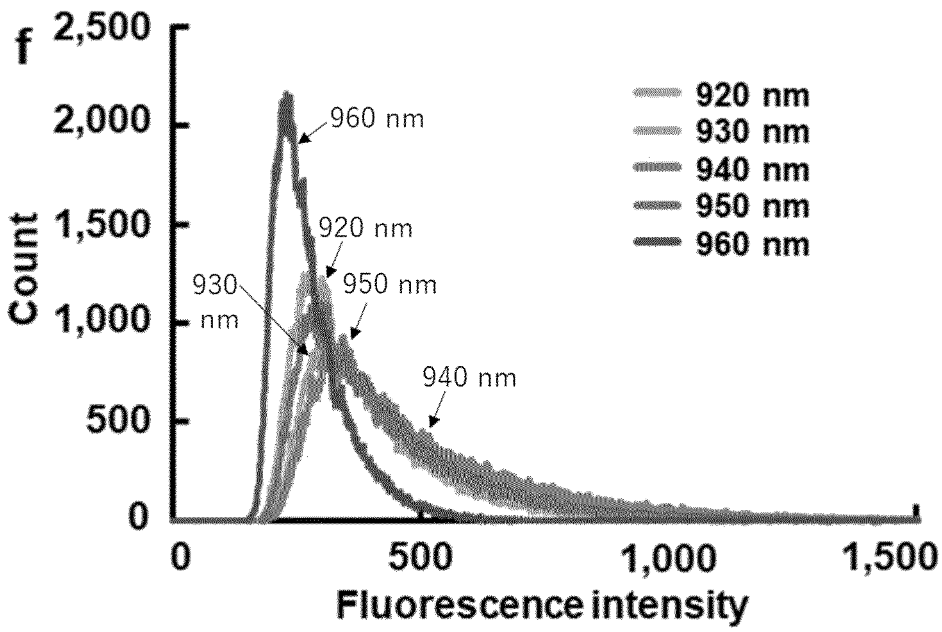
f
2,500
2,000
1,500
1,000
500
0
Count
0
500
1,000
1,500
Fluorescence intensity
920 nm
930 nm
940 nm
950 nm
960 nm
960 nm
920 nm
930
nm
950 nm
940 nm

[FIG. 3-3]

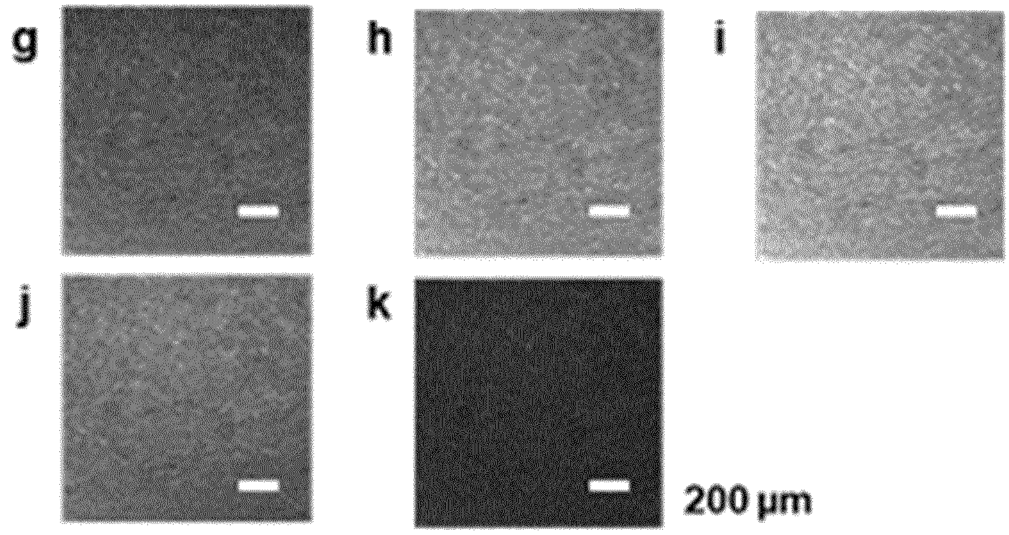
g
h
i
j
k
200 μm

[FIG. 3-4]

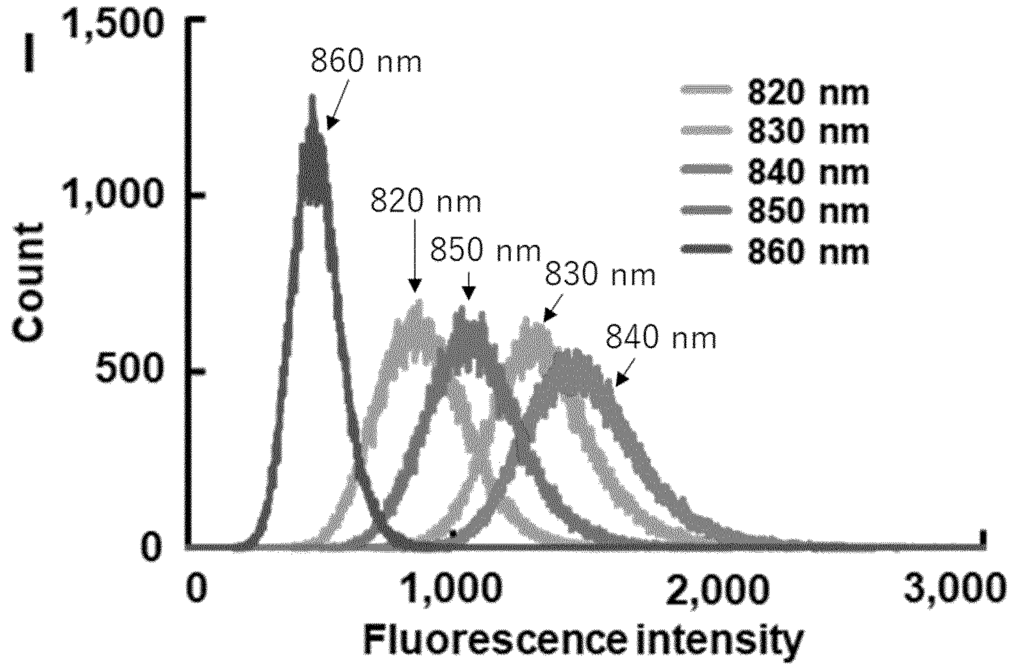
l
1,500
1,000
500
0
Count
0
1,000
2,000
3,000
Fluorescence intensity
860 nm
820 nm
850 nm
830 nm
840 nm
820 nm
830 nm
840 nm
850 nm
860 nm

[FIG. 3-5]

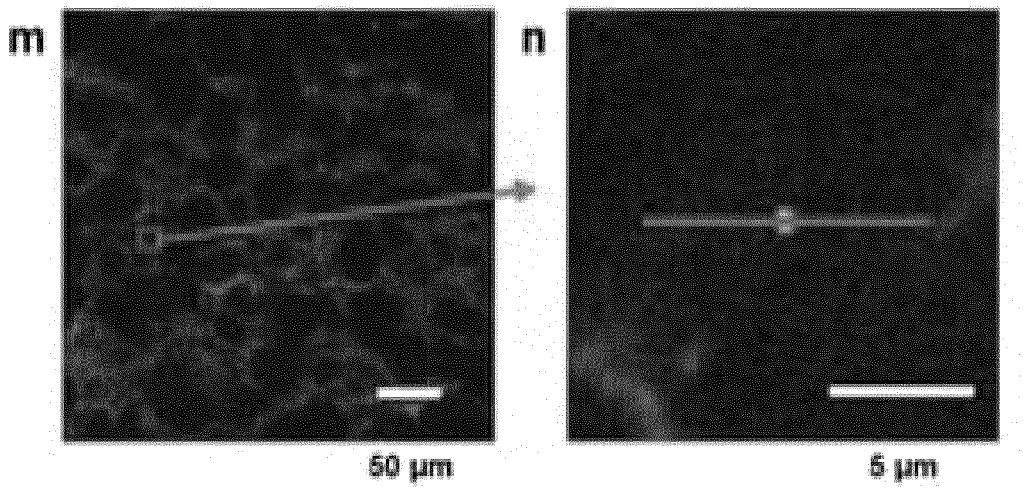
m
n
50 µm
5 µm

[FIG. 3-6]

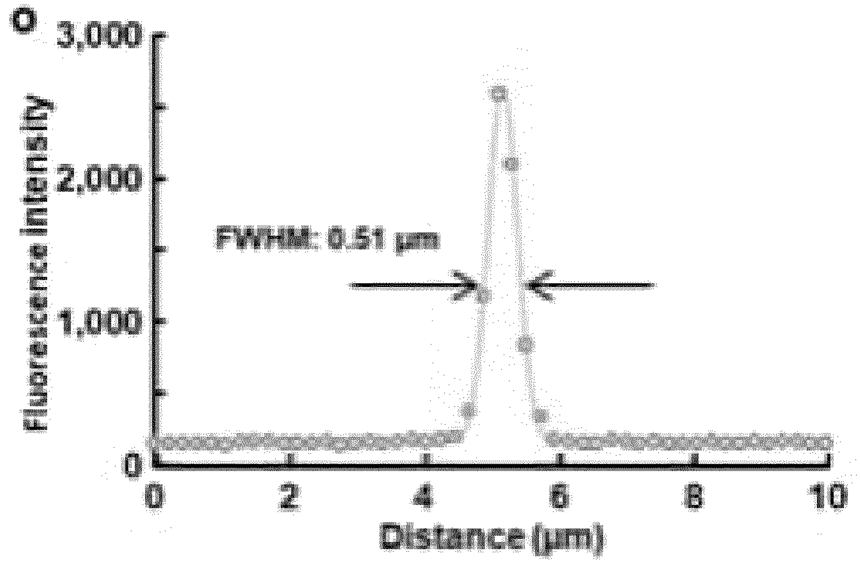
o
3,000
2,000
1,000
0
Fluorescence Intensity
FWHM: 0.51 µm
0
2
4
6
8
10
Distance (µm)

[FIG. 4-1]

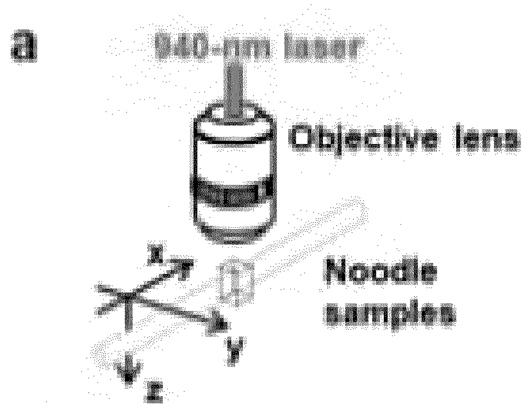
a
940-nm laser
Objective lens
x
y
z
Noodle
samples

[FIG. 4-2]

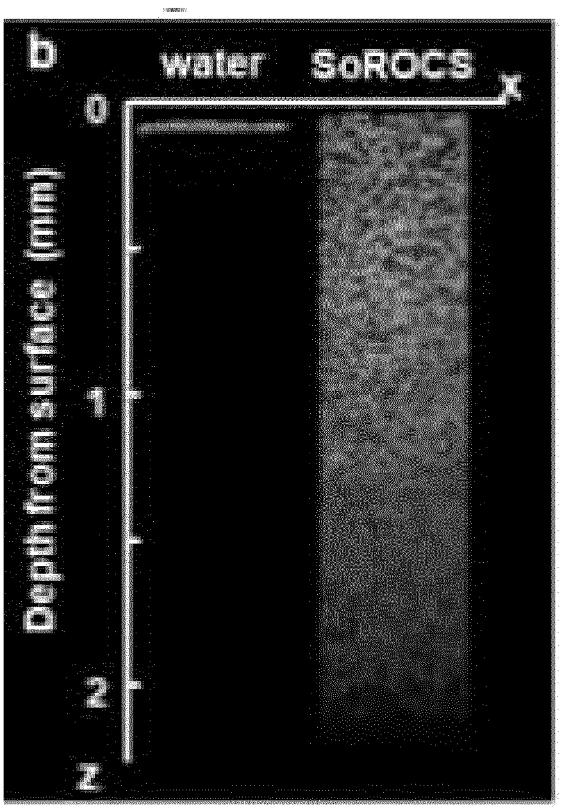
b
water
SoROCS
x
0
1
2
z
Depth from surface (mm)

[FIG. 4-3]

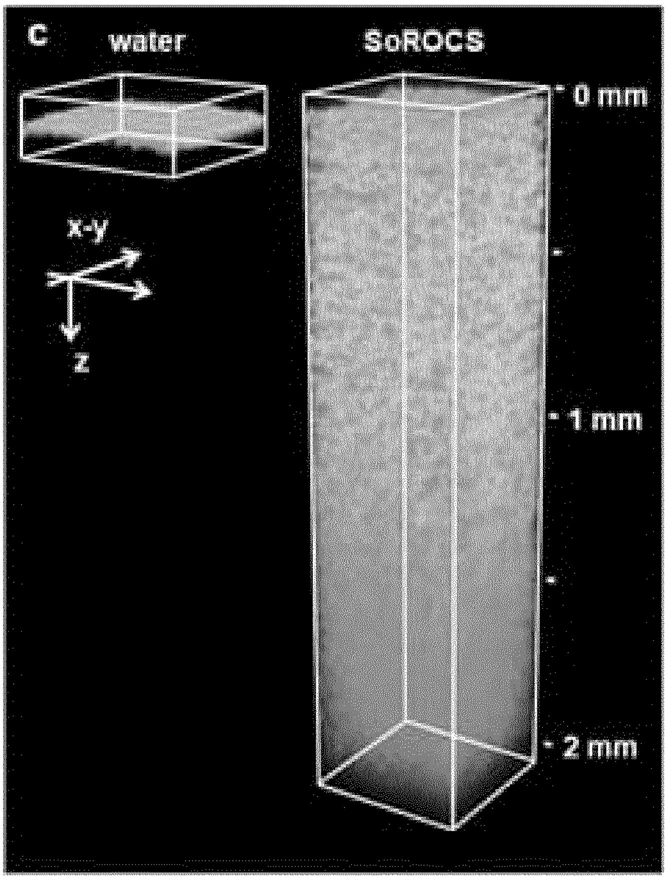
c
water
SoROCS
0 mm
1 mm
2 mm
x-y
z

[FIG. 4-4]

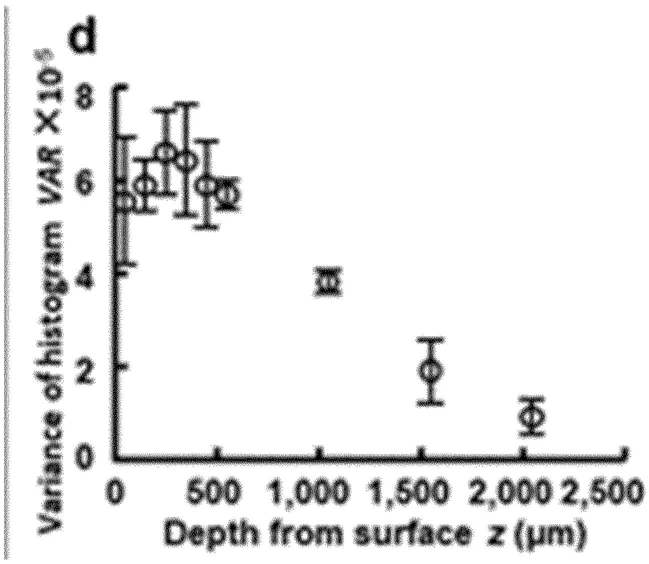
d
Variance of histogram VAR ×10^-5
0
2
4
6
8
0
500
1,000
1,500
2,000
2,500
Depth from surface z (μm)

[FIG. 4-5]

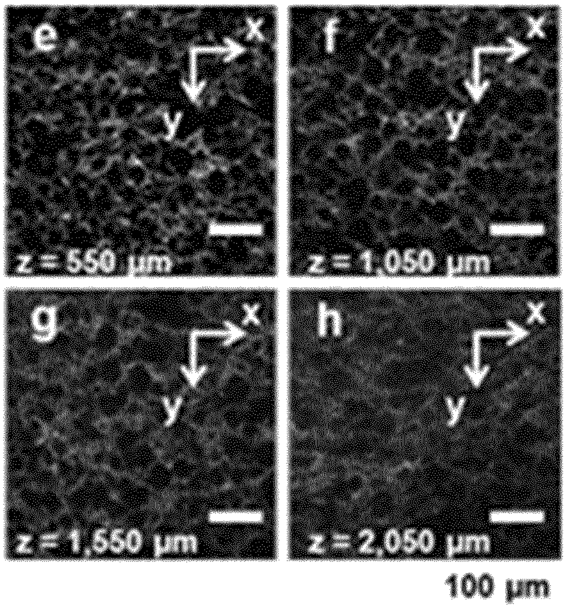


[FIG. 5-1]

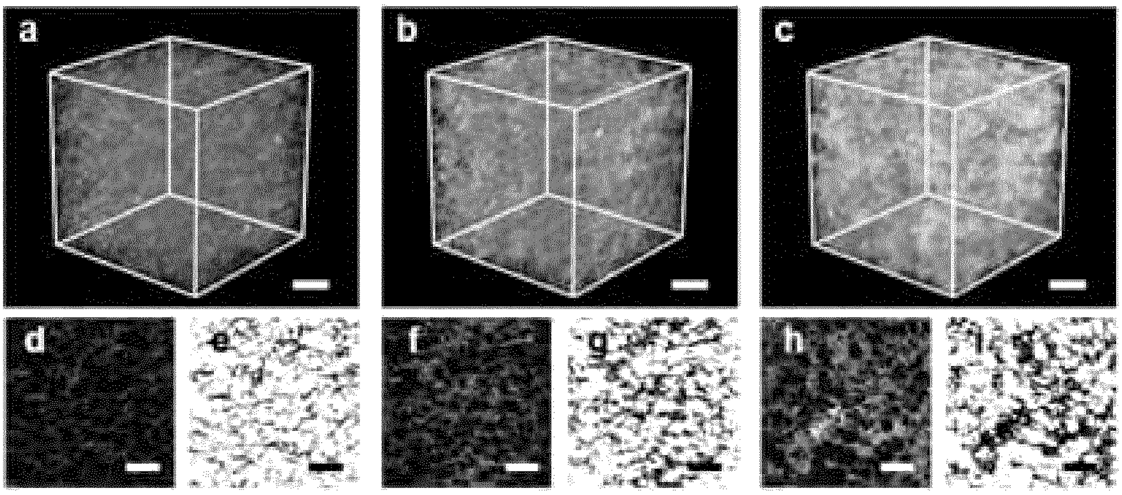


[FIG. 5-2]

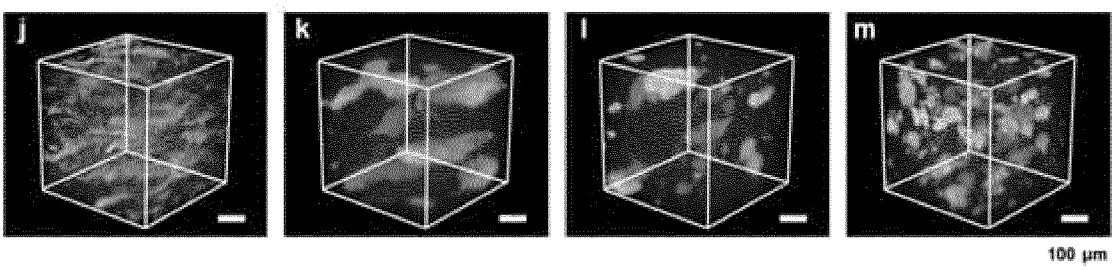

[FIG. 5-3]

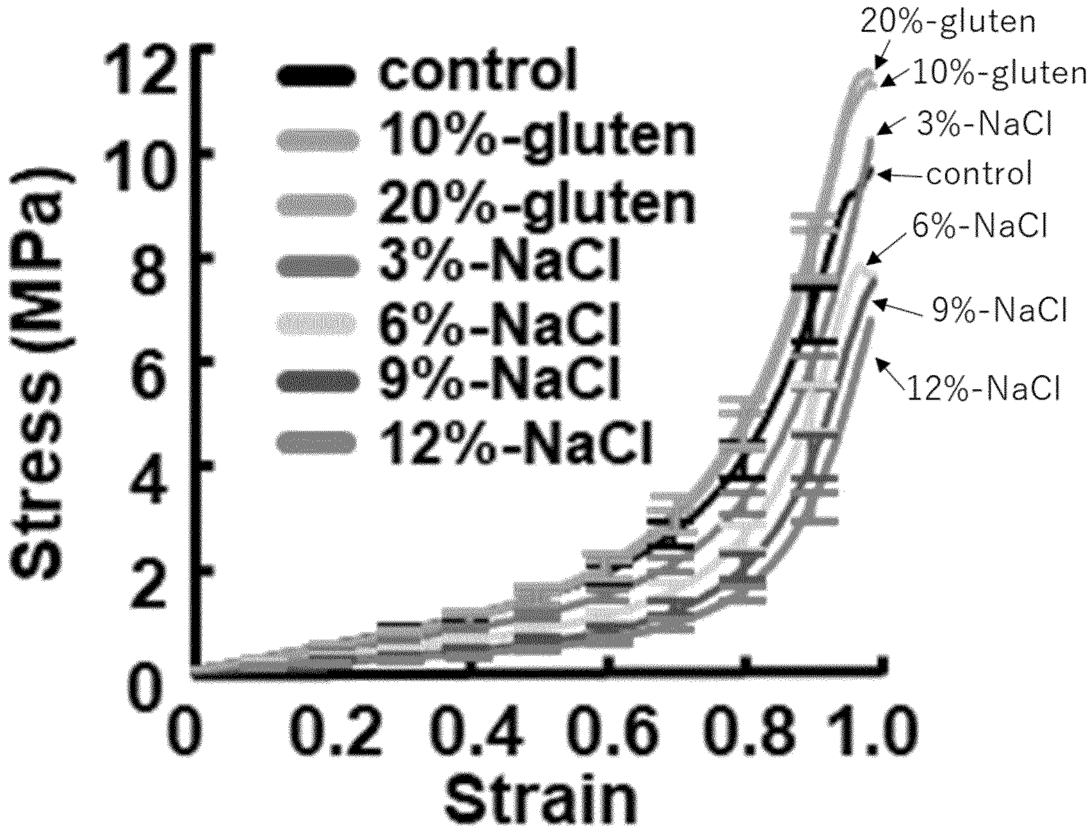
20%-gluten
10%-gluten
3%-NaCl
control
6%-NaCl
9%-NaCl
12%-NaCl
control
10%-gluten
20%-gluten
3%-NaCl
6%-NaCl
9%-NaCl
12%-NaCl
Stress (MPa)
12
10
8
6
4
2
0
0
0.2
0.4
0.6
0.8
1.0
Strain

[FIG. 6-1]

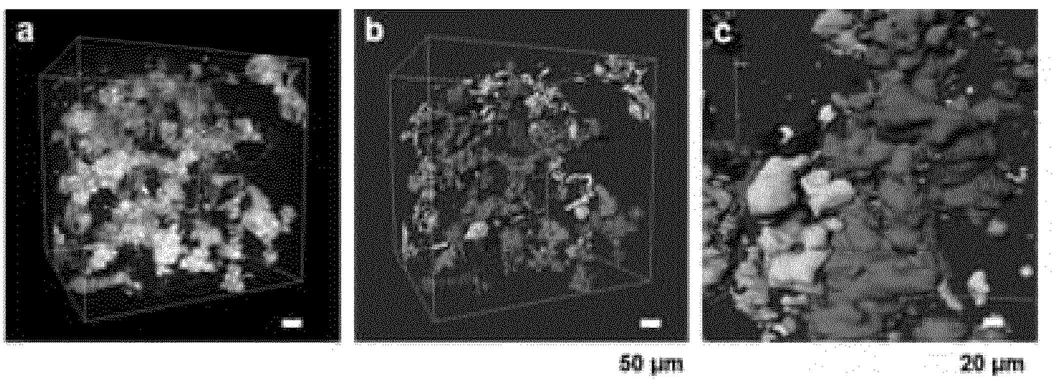
a
b
c
50 μm
20 μm

[FIG. 6-2]

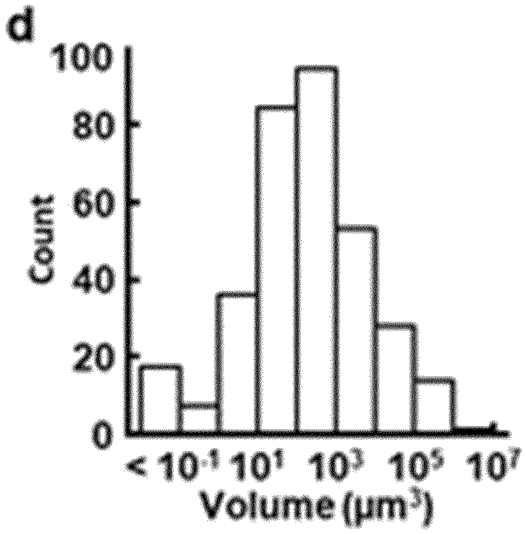
d
100
80
60
40
20
0
Count
< 10-1 10¹ 10³ 10⁵ 10⁷
Volume (μm³)

[FIG. 7-1]

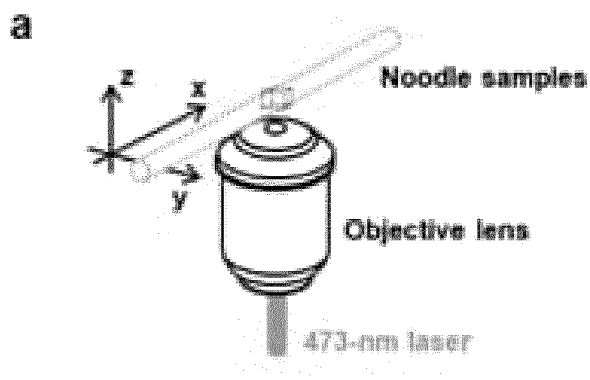
a
z
x
y
Noodle samples
Objective lens
473-nm laser

[FIG. 7-2]

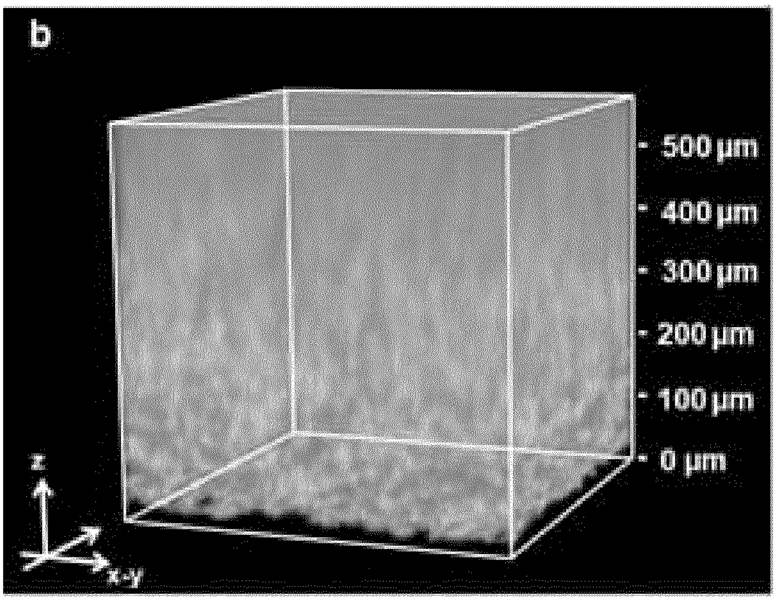
b
500 µm
400 µm
300 µm
200 µm
100 µm
0 µm
z
x-y

[FIG. 7-3]

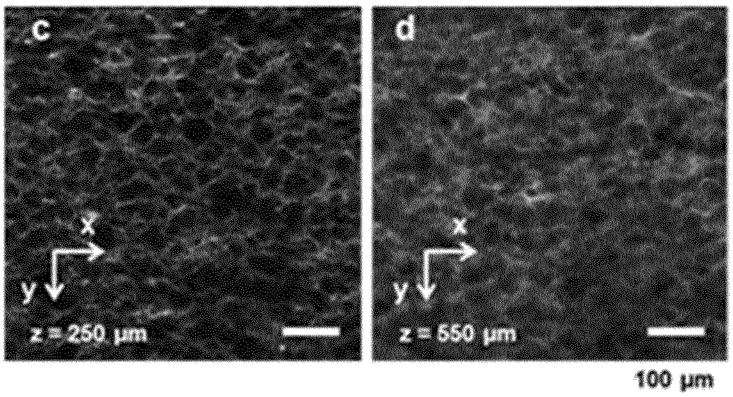
c
x
y
z = 250 µm
d
x
y
z = 550 µm
100 µm

[FIG. 7-4]

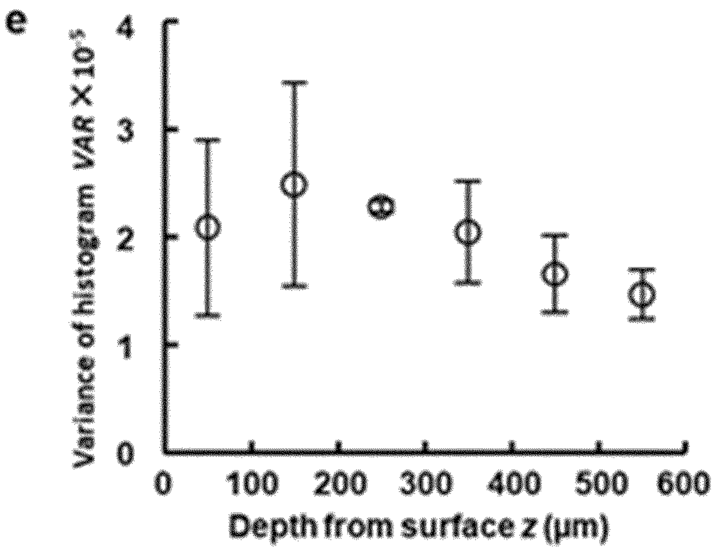
e
Variance of histogram VAR × 10⁻⁵
0
1
2
3
4
0
100
200
300
400
500
600
Depth from surface z (µm)

[FIG. 8]

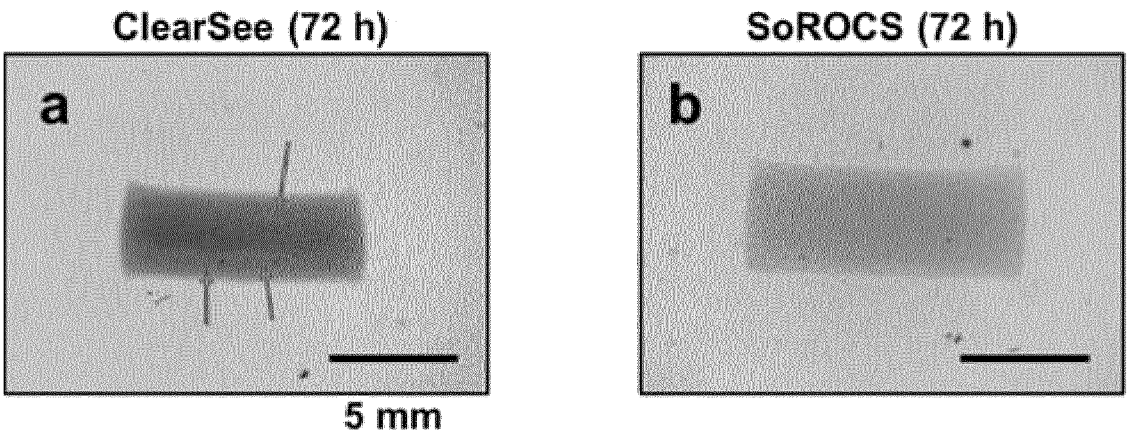
ClearSee (72 h)
SoROCS (72 h)
a
b
5 mm

[FIG. 9]

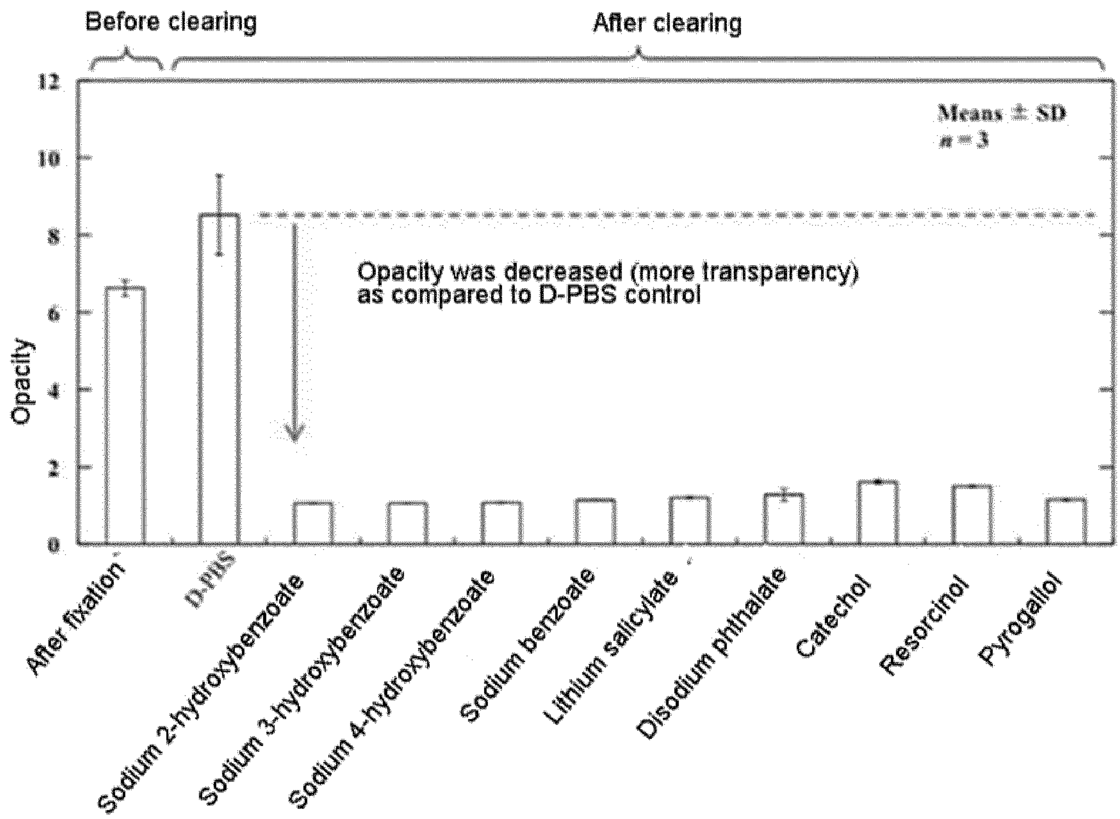
Before clearing
After clearing
Means ± SD
n = 3
Opacity
0
2
4
6
8
10
12
Opacity was decreased (more transparency)
as compared to D-PBS control
After fixation
D-PBS
Sodium 2-hydroxybenzoate
Sodium 3-hydroxybenzoate
Sodium 4-hydroxybenzoate
Sodium benzoate
Lithium salicylate
Disodium phthalate
Catechol
Resorcinol
Pyrogallol

## INTERNATIONAL SEARCH REPORT

International application No.
**PCT/JP2022/011135**

**A. CLASSIFICATION OF SUBJECT MATTER**

***G01N 1/30***(2006.01)i
FI: G01N1/30

According to International Patent Classification (IPC) or to both national classification and IPC

**B. FIELDS SEARCHED**

Minimum documentation searched (classification system followed by classification symbols)

G01N1/30

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | RICHARDSON, D. S. et al. Clarifying Tissue Clearing. Cell. 16 July 2015, 162(2), 246-257 table 1 | 1-27 |
| A | JP 2019-536005 A (RUTGERS, THE STATE UNIVERSITY OF NEW JERSEY) 12 December 2019 (2019-12-12) | 1-27 |
| A | JP 2010-535332 A (QIAGEN GMBH) 18 November 2010 (2010-11-18) | 1-27 |
| A | JP 2019-148601 A (VENTANA MEDICAL SYSTEMS, INC.) 05 September 2019 (2019-09-05) | 1-27 |
| A | JP 2015-049101 A (OLYMPUS CORP) 16 March 2015 (2015-03-16) | 1-27 |
| A | JP 2003-066035 A (KO, Anse) 05 March 2003 (2003-03-05) | 1-27 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 May 2022** | **31 May 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

INTERNATIONAL SEARCH REPORT

International application No.
PCT/JP2022/011135

**Box No. III Observations where unity of invention is lacking (Continuation of item 3 of first sheet)**

This International Searching Authority found multiple inventions in this international application, as follows:

(Invention 1) Claims 1-27
Claims 1-27 have the special technical feature of a reagent for transparentizing a carbohydrate- or protein-containing material, the reagent comprising benzene carboxylic acid compound and the like, and are thus classified as invention 1.

(Invention 2) Claim 28
Claim 28 cannot be said to share a same or corresponding technical feature with claim 1 classified as invention 1. Moreover, claim 28 is not substantially identical to or similarly closely related to any of the claims classified as invention 1.
Accordingly, claim 28 cannot be classified as invention 1.
In addition, claim 28 has the special technical feature of a decoloring agent containing 1,2-hexanediol, and is thus classified as invention 2.

(Invention 3) Claim 29
Claim 29 cannot be said to share a same or corresponding technical feature with claims 1-27 classified as invention 1 or claim 28 classified as invention 2. Moreover, claim 29 is not substantially identical to or similarly closely related to any of the claims classified as either invention 1 or invention 2.
Accordingly, claim 29 cannot be classified as either invention 1 or invention 2.
In addition, claim 29 has the special technical feature of a degreasing agent containing limonene, and is thus classified as invention 3.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-27**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2022/011135**

| Patent document cited in search report | Publication date (day/month/year) | Patent family member(s) | Publication date (day/month/year) |
|---|---|---|---|
| JP 2019-536005 A | 12 December 2019 | WO 2013/155064 A1 | |
| JP 2010-535332 A | 18 November 2010 | US 2010/0255571 A1<br>WO 2009/016254 A1<br>EP 2027922 A1<br>CN 101778672 A | |
| JP 2019-148601 A | 05 September 2019 | US 2016/0282239 A1<br>WO 2015/086484 A1<br>CA 2928900 A | |
| JP 2015-049101 A | 16 March 2015 | WO 2015/030164 A1 | |
| JP 2003-066035 A | 05 March 2003 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5967528 B
- JP 6044900 B


### Non-patent literature cited in the description


- **KURIHARA D. et al.** *Development,* 2015, vol. 142, 4168-4179